# EUROPEAN PATENT APPLICATION

(11) **EP 4 353 799 A1**
(43) Date of publication of application: **17.04.2024**
(21) Application number: 22837511.9
(22) Date of filing: 24.06.2022
(51) Int. Cl.: C09K 3/00, B01J 19/12, C02F 1/32, A61L 9/20

(54) **APPARATUS FOR TREATING FLUID WITH ULTRAVIOLET LIGHT**

(30) Priority: 08.07.2021 JP 2021113658; 17.09.2021 JP 2021152303; 10.12.2021 JP 2021201185; 11.05.2022 JP 2022078359
(71) Applicant: Nichia Corporation, Anan-shi, Tokushima 774-8601 (JP)
(72) Inventor: IMAI, Masahiro, Anan-shi, Tokushima 774-8601 (JP); MIYAZAWA, Masaaki, Anan-shi, Tokushima 774-8601 (JP); SANO, Hiroki, Anan-shi, Tokushima 774-8601 (JP); ONOZUKA, Katsuyuki, Anan-shi, Tokushima 774-8601 (JP)
(74) Representative: Winter, Brandl - Partnerschaft mbB
(86) International application number: PCT/JP2022/025389
(87) International publication number: WO 2023/282094

(57) **Abstract**

An ultraviolet light fluid treatment device that can enhance treatment effects is provided. The ultraviolet light fluid treatment device includes an inlet and an outlet of fluid; a flow channel connecting the inlet and the outlet and including a plurality of branch flow channels branching from the inlet, and a merged flow channel connected to a downstream side of each of the branch flow channels; a first light source configured to emit ultraviolet light to the merged flow channel; and a plurality of second light sources configured to emit the ultraviolet light to the plurality of respective branch flow channels.

## Description

### TECHNICAL FIELD

The present invention relates to an ultraviolet light fluid treatment device.

### BACKGROUND ART

For example, Patent Document 1 discloses a device that emits ultraviolet light from a light emitting element into a flow channel in which fluid flows.

### RELATED-ART DOCUMENTS

### PATENT DOCUMENTS

Patent Document 1: Japanese Laid-open Patent Publication No. 2018-140001

### SUMMARY OF THE INVENTION

### PROBLEM TO BE SOLVED BY THE INVENTION

It is an object of the present invention to provide an ultraviolet light fluid treatment device that can enhance a treatment effect.

### MEANS TO SOLVE THE PROBLEM

According to an aspect of the present invention, an ultraviolet light fluid treatment device includes an inlet and an outlet of fluid; a flow channel connecting the inlet and the outlet and including a plurality of branch flow channels branching from the inlet, and a merged flow channel connected to a downstream side of each of the branch flow channels; a first light source configured to emit ultraviolet light to the merged flow channel; and a plurality of second light sources configured to emit the ultraviolet light to the plurality of respective branch flow channels.

### EFFECTS OF THE INVENTION

According to the present invention, an ultraviolet light fluid treatment device that can enhance a treatment effect can be provided.

### BRIEF DESCRIPTION OF THE DRAWINGS

[FIG. 1] FIG. 1 is a perspective view of an ultraviolet light fluid treatment device according to a first embodiment of the present invention;
[FIG. 2] FIG. 2 is a cross-sectional view of the ultraviolet light fluid treatment device taken through line II-II of FIG. 1;
[FIG. 3] FIG. 3 is a cross-sectional view of an ultraviolet light fluid treatment device according to a second embodiment of the present invention;
[FIG. 4A] FIG. 4A is a perspective view illustrating an example of a light source according to an embodiment of the present invention;
[FIG. 4B] FIG. 4B is a perspective view illustrating another example of a light source according to an embodiment of the present invention;
[FIG. 5] FIG. 5 is a perspective view illustrating another example of a light source according to an embodiment of the present invention;
[FIG. 6] FIG. 6 is an exploded perspective view of the light source illustrated in FIG. 5;
[FIG. 7] FIG. 7 is a cross-sectional view of the ultraviolet light fluid treatment device, which illustrates a first example of fluid retention;
[FIG. 8] FIG. 8 is a perspective view of the ultraviolet light fluid treatment device, which illustrates the first example of fluid retention;
[FIG. 9] FIG. 9 is a cross-sectional view of the ultraviolet light fluid treatment device, which illustrates a second example of fluid retention;
[FIG. 10] FIG. 10 is a cross-sectional view of the ultraviolet light fluid treatment device, which illustrates a third example of fluid retention;
[FIG. 11] FIG. 11 is a cross-sectional view of the ultraviolet light fluid treatment device, which illustrates a fourth example of fluid retention;
[FIG. 12] FIG. 12 is a cross-sectional view of the ultraviolet light fluid treatment device, which illustrates a fifth example of fluid retention;
[FIG. 13] FIG. 13 is a cross-sectional view of the ultraviolet light fluid treatment device, which illustrates a sixth example of fluid retention;
[FIG. 14] FIG. 14 is a side view of the ultraviolet light fluid treatment device as viewed from the inlet side, which illustrates a seventh example of fluid retention;
[FIG. 15] FIG. 15 is a cross-sectional view of the ultraviolet light fluid treatment device taken through XV-XV of FIG. 14;
[FIG. 16] FIG. 16 is a cross-sectional view of the ultraviolet light fluid treatment device taken through XVI-XVI of FIG. 15;
[FIG. 17] FIG. 17 is a cross-sectional view of an ultraviolet light fluid treatment device according to a third embodiment of the present invention;
[FIG. 18] FIG. 18 is a cross-sectional view of an ultraviolet light fluid treatment device according to a fourth embodiment of the present invention;
[FIG. 19] FIG. 19 is a drawing illustrating partition members included in an ultraviolet light fluid treatment device according to a fifth embodiment of the present invention;
[FIG. 20] FIG. 20 is a perspective view of a light source including a photodetector in an ultraviolet light fluid treatment device according to a sixth embodiment of the present invention;
[FIG. 21] FIG. 21 is a first diagram illustrating the operation of the photodetector of FIG. 20; and
[FIG. 22] FIG. 22 is a second diagram illustrating the operation of the photodetector of FIG. 20.

### MODE FOR CARRYING OUT THE INVENTION

In the following, embodiments will be described with reference to the accompanying drawings. In the drawings, the same elements are denoted by the same reference numerals. Note that the drawings schematically illustrate the embodiments, and thus scales, intervals, positional relationships, and the like of members may be exaggerated, or illustration of some of the members may be omitted.

### [First Embodiment]

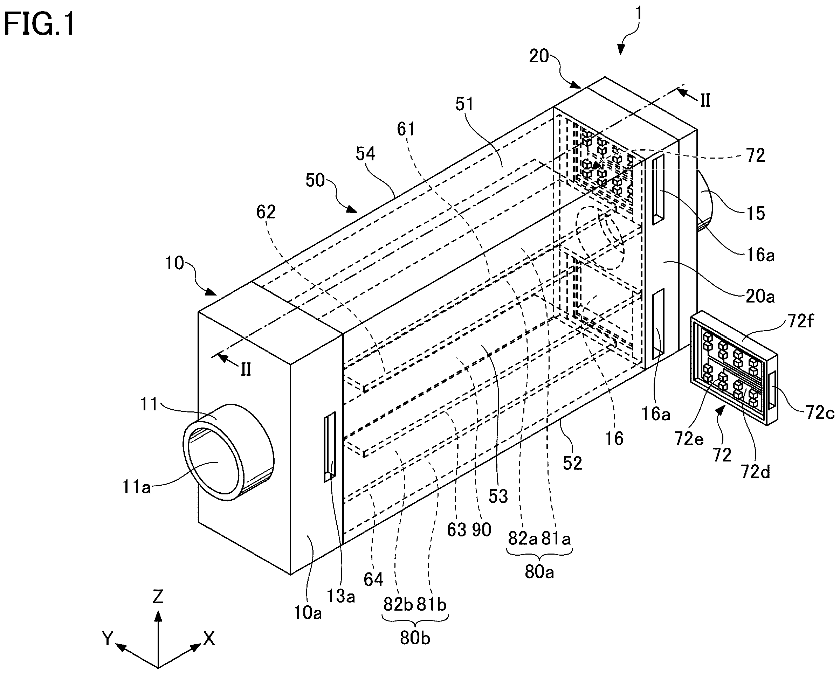

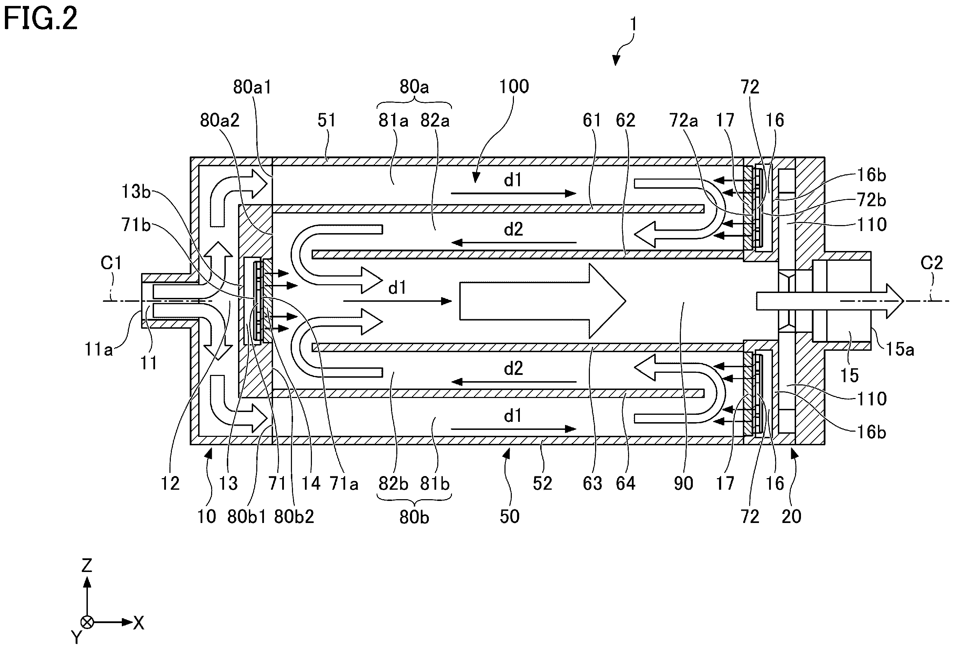

FIG. 1 is a perspective view of an ultraviolet light fluid treatment device 1 according to a first embodiment of the present invention. FIG. 2 is a cross-sectional view of the ultraviolet light fluid treatment device 1 taken through line II-II of FIG. 1. In FIG. 1 and FIG. 2, three axes orthogonal to each other are defined as an X-axis, a Y-axis, and a Z-axis. The cross section illustrated in FIG. 2 is parallel to the X-axis and the Z-axis, and is orthogonal to the Y-axis.

The ultraviolet light fluid treatment device 1 includes a first end portion 10, a second end portion 20, and an intermediate portion 50 located between the first end portion 10 and the second end portion 20. Further, the ultraviolet light fluid treatment device 1 includes a first light source 71 and a second light source 72. In the example illustrated in FIG. 1 and FIG. 2, the first light source 71 is disposed in the first end portion 10 and the second light source 72 is disposed in the second end portion 20.

The material of the first end portion 10, the second end portion 20, and the intermediate portion 50 is metal, and is, for example, stainless steel. The first end portion 10, the second end portion 20, and the intermediate portion 50 may be separated from one another, or may be integrated into one another.

In FIG. 2, the flows of fluid are indicated by bold arrows. The fluid such as liquid or gas flows into the first end portion 10 from the outside of the ultraviolet light fluid treatment device 1. The fluid further flows from the first end portion 10 through the intermediate portion 50 to the second end portion 20, and flows out from the second end portion 20 to the outside of the ultraviolet light fluid treatment device 1.

The first end portion 10 includes an inlet 11 of the fluid, an upstream flow channel 12, a first light source placement portion 13, and a first window 14.

The inlet 11 includes a hole leading from the outside of the ultraviolet light fluid treatment device 1 to the inside of the first end portion 10. An external pipe is connected to the inlet 11, and the fluid flows into the inlet 11 from the external pipe. The cross-sectional shape of the inlet 11 orthogonal to a direction in which the fluid flows is, for example, a circular shape. The inlet 11 includes an inlet port 11a formed as a circular opening, for example. A central axis C1 passing through the center of the circular cross-sectional shape of the inlet 11 is parallel to the X-axis direction.

The upstream flow channel 12 is connected to the inlet 11 within the first end portion 10. The upstream flow channel 12 branches into a plurality of flow channels from the inlet 11. In the example illustrated in FIG. 2, the upstream flow channel 12 branches into two flow channels from the inlet 11. For example, the upstream flow channel 12 branches from the inlet 11 in directions opposite to each other in the Z-axis direction that is orthogonal to the central axis C1.

The first light source placement portion 13 is formed as a space within the first end portion 10 in which the first light source 71 can be disposed. As illustrated in FIG. 1, a first opening 13a leading to the first light source placement portion 13 is formed in a lateral surface 10a of the first end portion 10. The first light source 71 can be detachably attached to the first light source placement portion 13 through the first opening 13a. The first light source placement portion 13 is formed as a space separated from each flow channel of the ultraviolet light fluid treatment device 1, and the first light source 71 is not exposed to the fluid and is protected from the fluid. For example, if the fluid is liquid, the first light source 71 does not require a waterproof structure. Further, the first light source 71 can be detached, replaced, and maintained while the fluid is flowing in the ultraviolet light fluid treatment device 1. The first light source placement portion 13 may be provided within the intermediate portion 50. In this case, the first opening 13a leading to the first light source placement portion 13 is formed in a third wall 53 or a fourth wall 54 of the intermediate portion 50 described later.

The first light source 71 emits ultraviolet light. The peak wavelength of the ultraviolet light emitted from the first light source 71 is, for example, 10 nm or more and 400 nm or less. The first light source 71 includes light emitting elements. As the light emitting elements, light emitting diodes (LEDs) or laser diodes (LDs) can be used, for example. As the first light source 71, a light emitting device in which light emitting elements are mounted on a wiring substrate, a light emitting device in which housings including light emitting elements are mounted on a wiring substrate, or the like can be used. The first light source 71 has a first surface 71a and a second surface 71b located opposite to the first surface 71a. The first surface 71a is a light emitting surface, and the ultraviolet light is emitted from the first surface 71a.

The first window 14 is disposed to face the first surface 71a of the first light source 71. The first surface 71a is located between the first window 14 and the second surface 71b of the first light source 71 in the X-axis direction, and a portion of the upstream flow channel 12 is located between the second surface 71b and the inlet 11 in the X-axis direction. The first window 14 is formed of a material having transmissivity with respect to the wavelength of the light emitted from the first light source 71. Examples of the material of the first window 14 include inorganic materials formed of at least one material selected from the group consisting of quartz glass, borosilicate glass, calcium fluoride glass, aluminoborosilicate glass, oxynitride glass, chalcogenide glass, and sapphire.

The second end portion 20 includes an outlet 15 of the fluid, a second light source placement portion 16, and a second window 17.

The outlet 15 includes a hole leading from the inside of the second end portion 20 to the outside of the ultraviolet light fluid treatment device 1. An external pipe is connected to the outlet 15. The fluid flowing in the ultraviolet light fluid treatment device 1 flows out from the outlet 15 into the external pipe. The cross-sectional shape of the outlet 15 orthogonal to the direction in which the fluid flows is, for example, a circular shape. The outlet 15 includes an outlet port 15a formed as a circular opening, for example.

A central axis C2 passing through the center of the circular cross-sectional shape of the outlet 15 preferably coincides with the central axis C1 of the inlet 11. Accordingly, the ultraviolet light fluid treatment device 1 can be easily connected to an intermediate portion of a straight pipe that is generally available.

The second light source placement portion 16 is formed as a space within the second end portion 20 in which the second light source 72 can be disposed. A plurality of second light sources 72 is disposed within the second end portion 20. In the present embodiment, for example, two second light sources 72 are disposed within the second end portion 20. Thus, two second light source placement portions 16 are formed within the second end portion 20. The outlet 15 is interposed between the two second light source placement portions 16 in the Z-axis direction.

As illustrated in FIG. 1, a second opening 16a leading to a corresponding second light source placement portion 16 is formed in a lateral surface 20a of the second end portion 20. A second light source 72 can be detachably attached to the corresponding second light source placement portion 16 through the second opening 16a. The second light source placement portion 16 is formed as a space separated from each flow channel of the ultraviolet light fluid treatment device 1, and the second light source 72 is not exposed to the fluid and is protected from the fluid. For example, if the fluid is liquid, the second light source 72 does not require a waterproof structure. Further, the second light source 72 can be detached, replaced, and maintained while the fluid is flowing in the ultraviolet light fluid treatment device 1. The second light source placement portion 16 may be disposed within the intermediate portion 50. In this case, the second opening 16a leading to the second light source placement portion 16 is formed in the third wall 53 or the fourth wall 54 of the intermediate portion 50 described later.

The second light source 72 emits ultraviolet light. As the second light source 72, the same light source as the first light source 71 can be used. As the second light source 72, a light source having an emission peak wavelength different from that of the first light source 71 may be used. The second light source 72 has a first surface 72a and a second surface 72b located opposite to the first surface 72a. The first surface 72a is a light emitting surface, and the ultraviolet light is emitted from the first surface 72a.

The second window 17 is disposed to face the first surface 72a of the second light source 72. The second window 17 is formed of a material having transmissivity with respect to the wavelength of the light emitted from the second light source 72. The second window 17 is formed of, for example, glass. The first surface 72a is located between the second window 17 and the second surface 72b of the second light source 72 in the X-axis direction.

As illustrated in FIG. 1, the second light source 72 includes, for example, a wiring substrate 72d, a plurality of light emitting elements 72e mounted on the wiring substrate 72d, and a housing 72f covering the wiring substrate 72d and the light emitting elements 72e. An insertion port 72c of a connector to be electrically connected to the wiring substrate 72d is formed in the housing 72f. The first light source 71 can be configured in the same manner as the second light source 72. The first light source 71 and the second light source 72 may both have a waterproof structure. In this case, the first light source placement portion 13 and the second light source placement portion 16 may be provided within a flow channel 100 of the ultraviolet light fluid treatment device 1. Further, light-transmissive members constituting the first window 14 and the second window 17 may be omitted. In addition, the ultraviolet light from the first light source 71 and the second light source 72 may be directly emitted from the first light source placement portion 13 and the second light source placement portion 16 into the flow channel 100.

The second end portion 20 further includes a downstream flow channel 110. The downstream flow channel 110 branches from the outlet 15 in the Z-axis direction, and leads to spaces each facing the second light source placement portion 16. A portion of the fluid, flowing into the outlet 15, can flow into the downstream flow channel 110 and cool the second light source 72 from the second surface 72b side. Accordingly, a decrease in light emission efficiency due to heat generation accompanying light emission of the second light source 72 can be reduced.

Further, the fluid flowing in the upstream flow channel 12 of the first end portion 10 can cool the first light source 71 from the second surface 71b side. Accordingly, a decrease in light emission efficiency due to heat generation accompanying light emission of the first light source 71 can be reduced.

In the example illustrated in FIG. 1, the intermediate portion 50 has four walls (a first wall 51, a second wall 52, the third wall 53, and the fourth wall 54) constituting a housing of the intermediate portion 50. The first wall 51 and the second wall 52 are separated from each other in the Z-axis direction. The third wall 53 and the fourth wall 54 are separated from each other in the Y-axis direction.

Further, the intermediate portion 50 includes a plurality of partition members 61 to 64 disposed in a space surrounded by the first wall 51, the second wall 52, the third wall 53, and the fourth wall 54. For example, four partition members (a first partition member 61, a second partition member 62, a third partition member 63, and a fourth partition member 64) are disposed in the intermediate portion 50.

Each of the first partition member 61, the second partition member 62, the third partition member 63, and the fourth partition member 64 is a plate member having a rectangular shape and extending in the X-axis direction. The first wall 51, the first partition member 61, the second partition member 62, the third partition member 63, the fourth partition member 64, and the second wall 52 are separated from one another in the Z-axis direction. Each of first flow channels 81a and 81b, second flow channels 82a and 82b, and a merged flow channel 90 may be configured with a tubular member.

In the Z-axis direction, the first partition member 61 is positioned between the first wall 51 and the second partition member 62, the second partition member 62 is positioned between the first partition member 61 and the third partition member 63, the third partition member 63 is positioned between the second partition member 62 and the fourth partition member 64, and the fourth partition member 64 is positioned between the third partition member 63 and the second wall 52.

The first partition member 61, the second partition member 62, the third partition member 63, and the fourth partition member 64 are interposed between the third wall 53 and the fourth wall 54 in the Y-axis direction. Both end portions in the Y-axis direction of each of the first partition member 61, the second partition member 62, the third partition member 63, and the fourth partition member 64 are supported by the third wall 53 and the fourth wall 54.

One end of the first partition member 61 is connected to the first end portion 10. The first partition member 61 extends from a connection portion between the first partition member 61 and the first end portion 10 toward the second end portion 20. The other end of the first partition member 61 is separated from the second end portion 20.

One end of the second partition member 62 is connected to the second end portion 20. The second partition member 62 extends from a connection portion between the second partition member 62 and the second end portion 20 toward the first end portion 10. The other end of the second partition member 62 is separated from the first end portion 10.

One end of the third partition member 63 is connected to the second end portion 20. The third partition member 63 extends from a connection portion between the third partition member 63 and the second end portion 20 toward the first end portion 10. The other end of the third partition member 63 is separated from the first end portion 10.

One end of the fourth partition member 64 is connected to the first end portion 10. The fourth partition member 64 extends from a connection portion between the fourth partition member 64 and the first end portion 10 toward the second end portion 20. The other end of the fourth partition member 64 is separated from the second end portion 20.

The intermediate portion 50 includes the flow channel 100 that connects the inlet 11 and the outlet 15 and is defined by the walls 51 to 54 and the partition members 61 to 64 described above. The flow channel 100 includes a plurality of branch flow channels 80a and 80b branching from the inlet 11, and the merged flow channel 90 connected to the downstream side of each of the branch flow channels 80a and 80b. In the example illustrated in FIG. 1, the merged flow channel 90 is interposed between the two branch flow channels 80a and 80b in the Z-axis direction.

At least one of the branch flow channels 80a and 80b include a first flow channel and a second flow channel. In the example illustrated in FIG. 1, the branch flow channel 80a includes the first flow channel 81a and the second flow channel 82a, and the branch flow channel 80b includes the first flow channel 81b and the second flow channel 82b.

The branch flow channel 80a includes the first flow channel 81a and the second flow channel 82a. The first flow channel 81a is disposed upstream of the second flow channel 82a, and the second flow channel 82a is disposed downstream of the first flow channel 81a. The term "upstream" refers to a side relatively close to the inlet 11 and the term "downstream" refers to a side relatively close to the outlet 15 in the flow channel from the inlet 11 toward the outlet 15.

The branch flow channel 80b includes the first flow channel 81b and the second flow channel 82b. The first flow channel 81b is disposed upstream of the second flow channel 82b, and the second flow channel 82b is disposed downstream of the first flow channel 81b.

The first flow channel 81a of the branch flow channel 80a is defined by the first wall 51, the first partition member 61, the third wall 53, and the fourth wall 54. The second flow channel 82a of the branch flow channel 80a is defined by the first partition member 61, the second partition member 62, the third wall 53, and the fourth wall 54.

The first flow channel 81b of the branch flow channel 80b is defined by the second wall 52, the fourth partition member 64, the third wall 53, and the fourth wall 54. The second flow channel 82b of the branch flow channel 80b is defined by the third partition member 63, the fourth partition member 64, the third wall 53, and the fourth wall 54.

One end of each of the first flow channels 81a and 81b is connected to the upstream flow channel 12 formed within the first end portion 10. The first flow channels 81a and 81b extend in a first direction d1 from respective connection portions connected to the upstream flow channel 12. The first direction d1 is, for example, a direction parallel to the X-axis direction. The fluid flows in each of the first flow channels 81a and 81b in the first direction d1. Further, the first direction d1 may be a direction that is inclined with respect to the X-axis direction.

The first flow channel 81a of the branch flow channel 80a is connected to the second flow channel 82a through a space between the first partition member 61 and the second end portion 20. The first flow channel 81b of the branch flow channel 80b communicates with the second flow channel 82b through a space between the fourth partition member 64 and the second end portion 20.

The second flow channels 82a and 82b extend in a direction different from the first direction d1 from respective portions communicating with the first flow channels 81a and 81b. The fluid flows in each of the second flow channels 82a and 82b in a second direction d2. In the present embodiment, the second direction d2 is a direction opposite to the first direction d1.

The first flow channels 81a and 81b, the second flow channels 82a and 82b, and the merged flow channel 90 are adjacent to one another in the Z-axis direction. The first flow channel 81a of the branch flow channel 80a is adjacent to the second flow channel 82a of the branch flow channel 80a with the first partition member 61 interposed therebetween. The first flow channel 81b of the branch flow channel 80b is adjacent to the second flow channel 82b of the branch flow channel 80b with the fourth partition member 64 interposed therebetween. The merged flow channel 90 is adjacent to the second flow channel 82a of the branch flow channel 80a with the second partition member 62 interposed therebetween, and is adjacent to the second flow channel 82b of the branch flow channel 80b with the third partition member 63 interposed therebetween. In the Z-axis direction, the two second flow channels 82a and 82b are positioned between the two first flow channels 81a and 81b, and the merged flow channel 90 is positioned between the two second flow channels 82a and 82b.

The second flow channel 82a of the branch flow channel 80a is connected to the merged flow channel 90 through a space between the second partition member 62 and the first end portion 10. The second flow channel 82b of the branch flow channel 80b is connected to the merged flow channel 90 through a space between the third partition member 63 and the first end portion 10. The merged flow channel 90 extends in the X-axis direction from a portion communicating with the two second flow channels 82a and 82b, and is connected to the outlet 15. The fluid flowing in the second flow channels 82a and 82b merges into the merged flow channel 90 and flows in the merged flow channel 90 in the first direction d1.

The first light source 71 is disposed at a position where the merged flow channel 90 can be irradiated with the ultraviolet light. For example, the first light source 71 is disposed in the first light source placement portion 13 provided in the first end portion 10. The first surface (light emitting surface) 71a of the first light source 71 faces, through the first window 14, a merging portion of the two second flow channels 82a and 82b into the merged flow channel 90. The ultraviolet light emitted from the first surface 71a of the first light source 71 travels from the merging portion of the second flow channels 82a and 82b into the merged flow channel 90.

One or more second light sources 72 are disposed at a position where one branch flow channel can be irradiated with the ultraviolet light. In the example illustrated in FIG. 2, two second light sources 72 are disposed at respective positions where the branch flow channels 80a and 80b can be irradiated with the ultraviolet light. For example, the two second light sources 72 are disposed in respective second light source placement portions 16 provided in the second end portion 20. The two second light sources 72 are disposed at positions where the first flow channels 81a and 81b and the second flow channels 82a and 82b can be irradiated with the ultraviolet light. In the present embodiment, one second light source 72 of the two second light sources 72 is disposed at a position facing, through a corresponding second window 17, a portion where the first flow channel 81a communicates with the second flow channel 82a in the branch flow channel 80a. The other second light source 72 is disposed at a position facing, through a corresponding second window 17, a portion where the first flow channel 81b communicates with the second flow channel 82b in the branch flow channel 80b. The ultraviolet light emitted from a first surface 72a of the one second light source 72 travels from the communication portion between the first flow channel 81a and the second flow channel 82a into the first flow channel 81a and the second flow channel 82a. The ultraviolet light emitted from a first surface 72a of the other second light source 72 travels from the communication portion between the first flow channel 81b and the second flow channel 82b into the first flow channel 81b and the second flow channel 82b.

Next, fluid treatment that uses the ultraviolet light fluid treatment device 1 according to the present embodiment will be described.

The ultraviolet light fluid treatment device 1 treats fluid such as liquid or gas by irradiating the fluid with ultraviolet light. For example, the ultraviolet light fluid treatment device 1 can treat water by irradiating the water with ultraviolet light so as to reduce bacteria and viruses in the water after the treatment as compared to before the treatment.

The inlet 11 is connected to the external upstream pipe directly or via a joint member. The outlet 15 is connected to the external downstream pipe directly or via a joint member. The fluid flowing through the external upstream pipe flows into the inlet 11, and is separated into two at the upstream flow channel 12. One portion of the fluid separated into two flows into the first flow channel 81a of the branch flow channel 80a, and the other portion of the fluid flows into the first flow channel 81b of the branch flow channel 80b.

The fluid flowing into the first flow channels 81a and 81b flows in each of the first flow channels 81a and 81b in the first direction d1, and then flows into the second flow channels 82a and 82b at the ends, near the second end portion 20, of the first flow channels 81a and 81b. The fluid flowing into the second flow channels 82a and 82b flows in each of the second flow channels 82a and 82b in the second direction d2. The fluid flowing in the first flow channels 81a and 81b and the second flow channels 82a and 82b is irradiated with the ultraviolet light from the second light sources 72.

The fluid flowing in the second flow channels 82a and 82b in the second direction d2 flows into the merged flow channel 90. The fluid flowing into the merged flow channel 90 flows in the merged flow channel 90 in the first direction d1. The fluid flowing in the merged flow channel 90 is irradiated with the ultraviolet light from the first light source 71. The fluid flowing in the merged flow channel 90 flows out from the outlet 15 to the external pipe connected to the outlet 15.

According to the present embodiment, the fluid flowing into the inside of the ultraviolet light fluid treatment device 1 from the inlet 11 is separated one or more times, merges again, and flows out from the outlet 15. Accordingly, the length of the flow channel in which the fluid flows from the external upstream pipe connected to the inlet 11 to the external downstream pipe connected to the outlet 15 can be increased, as compared to when the fluid flows from the inlet 11 to the outlet 15 without being separated. In addition, the fluid flowing in the branch flow channels 80a and 80b is irradiated with the ultraviolet light from the second light sources 72, and the fluid flowing from the branch flow channels 80a and 80b into the merged flow channel 90 and flows in the merged flow channel 90 is irradiated with the ultraviolet light from the first light source 71. Accordingly, the integrated luminance of the ultraviolet light emitted to the fluid flowing in the ultraviolet light fluid treatment device 1 can be increased, and an effect of treating the fluid with the ultraviolet light can be enhanced.

The first direction d1 in which the fluid flows in the first flow channels 81a and 81b is different from the second direction d2 in which the fluid flows in the second flow channels 82a and 82b. Thus, the lengths of the branch flow channels 80a and 80b can be increased while suppressing an increase in the dimension of the flow channel 100 between the inlet 11 and the outlet 15 (the dimension of the flow channel 100 in the X-axis direction). Further, the first direction d1 and the second direction d2 are opposite to each other, and thus the lengths of the branch flow channels 80a and 80b can be increased while suppressing an increase in the dimensions of the flow channel 100 in the X-axis direction and the Z-axis direction.

The flow channels of the flow channel 100 preferably overlap one another only in one direction (in the present embodiment, in the Z-axis direction) in a cross-sectional view. With this configuration, the size of the ultraviolet light fluid treatment device 1 can be easily reduced as compared to a configuration in which flow channels of a flow channel are arranged concentrically one above another. Further, with a configuration in which flow channels are arranged concentrically one above another, annular members defining the flow channels are separated, and thus parts management efficiency and assembly efficiency would be decreased. Conversely, in the present embodiment, both end portions of each of the partition members 61 to 64 in the Y-axis direction are supported by the third wall 53 and the fourth wall 54 constituting part of the housing of the intermediate portion 50, and thus the partition members 61 to 64 can be integrated.

The branch flow channel 80a does not necessarily include the two flow channels (the first flow channel 81a and the second flow channel 82a), and the branch flow channel 80b does not necessarily include the two flow channels (the first flow channel 81b and the second flow channel 82b). Each of the branch flow channels 80a and 80b may include one flow channel or three or more flow channels. As the number of flow channels included in each of the branch flow channels 80a and 80b increases, the lengths of the branch flow channels 80a and 80b increase. Thus, the integrated luminance of the ultraviolet light emitted to the fluid flowing in the branch flow channels 80a and 80b can be increased. As the number of flow channels included in each of the branch flow channels 80a and 80b decreases, pressure loss of the fluid flowing in the ultraviolet light fluid treatment device 1 can be reduced.

If each of the branch flow channels 80a and 80b includes a plurality of flow channels, the fluid flowing in the branch flow channels 80a and 80b can be treated by irradiating at least one of the plurality of flow channels included in each of the branch flow channels 80a and 80b with the ultraviolet light from each of the second light sources 72. By causing the second light sources 72 to emit the ultraviolet light to two or more or all of the plurality of flow channels included in each of the branch flow channels 80a and 80b, the effect of treating the fluid, flowing in the branch flow channels 80a and 80b, with the ultraviolet light can be further enhanced.

If a vortex or turbulence is generated in the flow channel 100, pressure loss of the fluid tends to occur. For this reason, in order to reduce pressure loss of the fluid, a flow velocity difference or a flow rate difference between the fluid flowing from the branch flow channel 80a into the merged flow channel 90 and the fluid flowing from the branch flow channel 80b into the merged flow channel 90 is preferably reduced.

For example, the cross-sectional shape of each of the first flow channels 81a and 81b in a direction orthogonal to the first direction d1 in which the fluid flows is a rectangular shape. The cross-sectional shape of each of the second flow channels 82a and 82b in a direction orthogonal to the second direction d2 in which the fluid flows is a rectangular shape. The cross-sectional shape of the merged flow channel 90 in a direction orthogonal to the first direction d1 in which the fluid flows is a rectangular shape.

Further, the cross-sectional area of the first flow channel 81a in the direction orthogonal to the first direction d1 in which the fluid flows, the cross-sectional area of the first flow channel 81b in the direction orthogonal to the first direction d1 in which the fluid flows, the cross-sectional area of the second flow channel 82a in the direction orthogonal to the second direction d2 in which the fluid flows, and the cross-sectional area of the second flow channel 82b in the direction orthogonal to the second direction d2 in which the fluid flows are the same. Therefore, the cross-sectional area of the branch flow channel 80a in the direction orthogonal to the directions in which the fluid flows is the same as the cross-sectional area of the branch flow channel 80b in the direction orthogonal to the directions in which the fluid flows.

Further, the length from an upstream end 80a1 to a downstream end 80a2 of the branch flow channel 80a is the same as the length from an upstream end 80b1 to a downstream end 80b2 of the branch flow channel 80b.

Therefore, according to the present embodiment, a flow velocity difference or a flow rate difference between the fluid flowing from the branch flow channel 80a into the merged flow channel 90 and the fluid flowing from the branch flow channel 80b into the merged flow channel 90 can be reduced. Accordingly, pressure loss of the fluid due to a vortex or turbulence generated in the flow channel 100 can be reduced.

Further, the cross-sectional area of the first flow channel 81a of the branch flow channel 80a orthogonal to the direction in which the fluid flows is greater than or equal to the cross-sectional area of the inlet 11 orthogonal to the direction in which the fluid flows. For example, the cross-sectional area of the first flow channel 81a orthogonal to the direction in which the fluid flows is the same as the cross-sectional area of the inlet 11 orthogonal to the direction in which the fluid flows. The cross-sectional area of the first flow channel 81b of the branch flow channel 80b orthogonal to the direction in which the fluid flows is greater than or equal to the cross-sectional area of the inlet 11 orthogonal to the direction in which the fluid flows. For example, the cross-sectional area of the first flow channel 81b orthogonal to the direction in which the fluid flows is the same as the cross-sectional area of the inlet 11 orthogonal to the direction in which the fluid flows. Accordingly, the flow velocity of the fluid separated from the inlet 11 and flowing into the first flow channels 81a and 81b can be set to one-half or less of the flow velocity of the fluid flowing in the inlet 11. By reducing the flow velocity of the fluid flowing in the first flow channels 81a and 81b, the integrated illuminance of the ultraviolet light emitted from the second light sources 72 to the fluid flowing in the first flow channels 81a and 81b can be increased, and the effect of treating the fluid, flowing in the first flow channels 81a and 81b, with the ultraviolet light can be enhanced.

Further, the cross-sectional area of the second flow channel 82a of the branch flow channel 80a orthogonal to the direction in which the fluid flows is greater than or equal to the cross-sectional area of the inlet 11 orthogonal to the direction in which the fluid flows. For example, the cross-sectional area of the second flow channel 82a orthogonal to the direction in which the fluid flows is the same as the cross-sectional area of the inlet 11 orthogonal to the direction in which the fluid flows. The cross-sectional area of the second flow channel 82b of the branch flow channel 80b orthogonal to the direction in which the fluid flows is greater than or equal to the cross-sectional area of the inlet 11 orthogonal to the direction in which the fluid flows. For example, the cross-sectional area of the second flow channel 82b orthogonal to the direction in which the fluid flows is the same as the cross-sectional area of the inlet 11 orthogonal to the direction in which the fluid flows. Accordingly, the flow velocity of the fluid separated from the inlet 11, flowing into the first flow channels 81a and 81b, and then flowing in the second flow channels 82a and 82b can be set to one-half or less of the flow velocity of the fluid flowing in the inlet 11. By reducing the flow velocity of the fluid flowing in the second flow channels 82a and 82b, the integrated illuminance of the ultraviolet light emitted from the second light sources 72 to the fluid flowing in the second flow channels 82a and 82b can be increased, and the effect of treating the fluid, flowing in the second flow channels 82a and 82b, with the ultraviolet light can be enhanced.

The fluid from each of the second flow channels 82a and 82b of the two branch flow channels 80a and 80b merges and flows into the merged flow channel 90. Therefore, in order to reduce the flow velocity of the fluid flowing in the merged flow channel 90, the cross-sectional area of the merged flow channel 90 in the direction orthogonal to the direction in which the fluid flows is preferably set to be greater than the cross-sectional areas of the second flow channels 82a and 82b in the direction orthogonal to the direction in which the fluid flows. Accordingly, the integrated illuminance of the ultraviolet light emitted from the first light source 71 to the fluid flowing in the merged flow channel 90 can be increased, and effects of treating the fluid, flowing in the merged flow channel 90, with the ultraviolet light can be enhanced.

For example, the cross-sectional area of the merged flow channel 90 orthogonal to the direction in which the fluid flows is equal to a sum of the cross-sectional area of the second flow channel 82a orthogonal to the direction in which the fluid flows and the cross-sectional area of the second flow channel 82b orthogonal to the direction in which the fluid flows. Therefore, the flow velocity of the fluid flowing in each of the second flow channels 82a and 82b can be approximately the same as the flow velocity of the fluid flowing in the merged flow channel 90. By achieving uniform flow velocities or reducing a change in flow velocities, pressure loss of the fluid due to a vortex or turbulence can be reduced.

The merged flow channel 90 is not necessarily disposed between the branch flow channels 80a and 80b. For example, the two branch flow channels 80a and 80b illustrated in FIG. 2 may be disposed on the first wall 51 side (in FIG. 2, on the upper side) of the merged flow channel 90 in the Z-axis direction, or may be disposed on the second wall 52 side (in FIG. 2, on the lower side) of the merged flow channel 90 in the Z-axis direction. For example, the two branch flow channels 80a and 80b disposed on the first wall 51 side or the second wall 52 side of the merged flow channel 90 may be adjacent to each other in the Y-axis direction.

### [Second Embodiment]

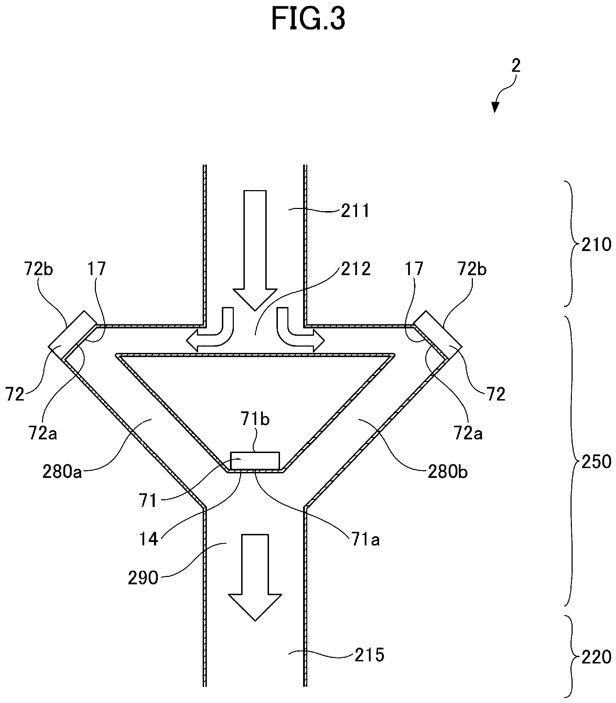

FIG. 3 is a cross-sectional view of an ultraviolet light fluid treatment device 2 according to a second embodiment of the present invention.

The ultraviolet light fluid treatment device 2 includes a first end portion 210, a second end portion 220, and an intermediate portion 250, the first light source 71, and the two second light sources 72. The first end portion 210 includes an inlet 211, and the second end portion 220 includes an outlet 215. The intermediate portion 250 is located between the first end portion 210 and the second end portion 220 in a direction from the inlet 211 toward the outlet 215.

The intermediate portion 250 includes an upstream flow channel 212 connected to the inlet 211, two branch flow channels 280a and 280b connected to the upstream flow channel 212, and a merged flow channel 290 connected to the outlet 215 side of each of the branch flow channels 280a and 280b.

In FIG. 3, the flows of fluid are indicated by bold arrows. An external upstream pipe is connected to the inlet 211, and the fluid flows into the inlet 211 from the external upstream pipe. The upstream flow channel 212 branches into two flow channels from the inlet 211. The branch flow channels 280a and 280b are connected to the two respective flow channels branching from the upstream flow channel 212. The fluid entering from the inlet 211 flows into the two branch flow channels 280a and 280b through the upstream flow channel 212. The outlet 215 side of each of the two branch flow channels 280a and 280b is connected to the merged flow channel 290. The fluid flowing in the two branch flow channels 280a and 280b merges and flows into the merged flow channel 290. An external downstream pipe is connected to the outlet 215, and the fluid flowing in the merged flow channel 290 flows out from the outlet 215 to the external downstream pipe.

In the example illustrated in FIG. 3, the first light source 71 is disposed in the intermediate portion 250. For example, the first surface (light emitting surface) 71a of the first light source 71 faces, through the first window 14, a portion where the two branch flow channels 280a and 280b are connected to the merged flow channel 290. The first light source 71 can emit ultraviolet light into the merged flow channel 290 in a direction from the inlet 211 toward the outlet 215.

One second light source 72 of the two second light sources 72 is disposed at a position where the branch flow channel 280a of the two branch flow channels 280a and 280b can be irradiated with ultraviolet light. The other second light source 72 is disposed at a position where the branch flow channel 280b can be irradiated with ultraviolet light. The first surface (light emitting surface) 72a of the one second light source 72 of the two second light sources 72 faces the branch flow channel 280a through the second window 17, and the first surface (light emitting surface) 72a of the other second light source 72 faces the branch flow channel 280b through the second window 17. The one second light source 72 can emit the ultraviolet light into the branch flow channel 280a in a direction from the inlet 211 toward the outlet 215. The other second light source 72 can emit the ultraviolet light into the branch flow channel 280b in a direction from the inlet 211 toward the outlet 215.

In the ultraviolet light fluid treatment device 2 according to the second embodiment, the fluid flowing into the inside of the ultraviolet light fluid treatment device 2 from the inlet 211 is separated one or more times, merges again, and flows out from the outlet 215. Accordingly, the length of the flow channel in which the fluid flows from the external upstream pipe connected to the inlet 211 to the external downstream pipe connected to the outlet 215 can be increased, as compared to when the fluid flows from the inlet 211 to the outlet 215 without being separated. In addition, the fluid flowing in the branch flow channels 280a and 280b is irradiated with the ultraviolet light from the second light sources 72, and the fluid flowing from the branch flow channels 280a and 280b into the merged flow channel 290 and flowing in the merged flow channel 290 is irradiated with the ultraviolet light from the first light source 71. Accordingly, the integrated luminance of the ultraviolet light emitted to the fluid flowing in the ultraviolet light fluid treatment device 2 can be increased, and the effect of treating the fluid with the ultraviolet light can be enhanced.

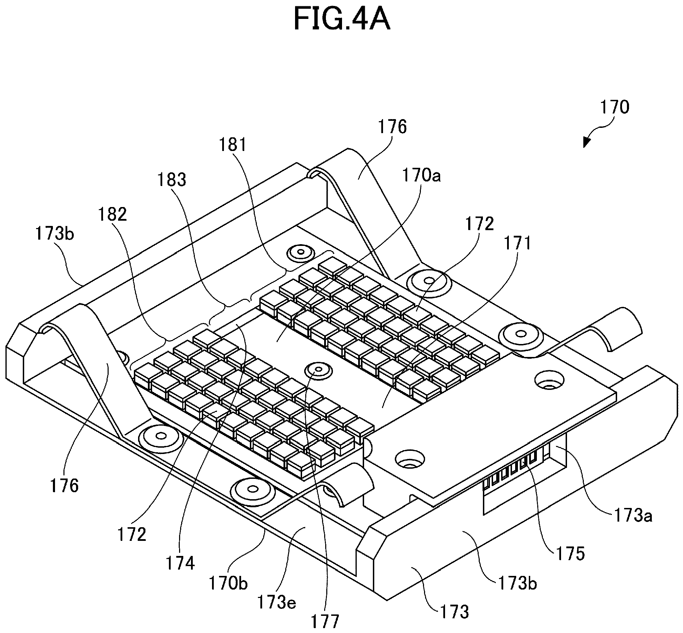

A light source 170 illustrated in FIG. 4A can be used as each of the first light source and the second light sources described above.

The light source 170 includes a wiring substrate 171 and a plurality of light emitting elements. The light emitting elements are mounted on the surface of the wiring substrate 171. The shape of the wiring substrate 171 in a plan view as viewed from the surface side of the wiring substrate 171 is, for example, a rectangle, and the center of the wiring substrate 171 is positioned at the intersection of two diagonal lines of the rectangle. The wiring substrate 171 has a first region 181 and a second region 182. The first region 181 and the second region 182 are arranged side by side in one direction of the wiring substrate 171. The wiring substrate 171 can further have a third region 183. The third region 183 is located between the first region 181 and the second region 182 in a plane parallel to the surface of the wiring substrate 171. The third region 183 includes the center of the wiring substrate 171. If the third region 183 is not provided, the center of the wiring substrate 171 is located, for example, at the boundary between the first region 181 and the second region 182. The first region 181 or the second region 182 may include the center of the wiring substrate 171.

The width of the third region 183 in the direction in which the first region 181, the third region 183, and the second region 182 are aligned is preferably the same as or greater than the thickness of the first partition member 61 and the fourth partition member 64.

In the example illustrated in FIG. 4A, a plurality of housings 172 is disposed in the first region 181. A plurality of housings 172 is disposed in the second region 182. The housings 172 each includes at least one light emitting element. Further, each of the housings 172 can include a lens disposed on the at least one light emitting element. Alternatively, light emitting elements not housed in the housings 172 may be disposed in the first region 181 and the second region 182. No light emitting element is disposed in the third region 183.

The light source 170 can include a holding member 173 that holds the wiring substrate 171. The holding member 173 has a surface 173e on which the wiring substrate 171 is mounted, and a surface located opposite to the surface 173e. The wiring substrate 171 is fixed to the surface 173e of the holding member 173 by, for example, a screw, an adhesive, or the like. A surface on which the housings 172 including the light emitting elements are mounted is a first surface 170a of the light source 170, and a surface located opposite to the surface 173e of the holding member 173 is a second surface 170b of the light source 170. The holding member 173 includes a wall portion 173b on the first surface 170a side of the light source 170. The wall portion 173b covers the end portion of the wiring substrate 171. For example, two wall portions 173b are disposed to sandwich the wiring substrate 171 in a plan view as viewed from the first surface 170a.

In the light source 170, wiring 174 electrically connected to the light emitting elements can be disposed on the surface of the wiring substrate 171. Further, a connector 175 electrically connected to the wiring 174 can be disposed on the surface of the wiring substrate 171. An insertion port 173a exposing the connector 175 from the holding member 173 is disposed in one of the wall portions 173b of the holding member 173.

A spring member 176 is disposed on the first surface 170a side of the light source 170. The spring member 176 is, for example, a metal leaf spring. For example, two spring members 176 sandwich the wiring substrate 171 in a plan view as viewed from the first surface 170a, and are fixed to the holding member 173.

The light source 170 can used as the first light source and disposed in the first light source placement portion 13 illustrated in FIG. 2. The first surface 170a of the light source 170 disposed in the first light source placement portion 13 faces the first window 14. The ultraviolet light from the first surface 170a is emitted through the first window 14 to the fluid flowing in the merged flow channel 90.

The light source 170 is disposed in the first light source placement portion 13 with the spring members 176 being elastically deformed from a natural state. The spring members 176 disposed on the first surface 170a side contact the first window 14. The restoring force of the spring members 176 causes the light source 170 to be preloaded toward a first partition wall 13b that partitions the upstream flow channel 12 and the first light source placement portion 13, and the second surface 170b is pressed against the first partition wall 13b. Accordingly, the cooling efficiency of the light source 170 by the fluid flowing in the upstream flow channel 12 can be increased.

Further, the light source 170 can be used as each of the second light sources and disposed in each of the second light source placement portions 16 illustrated in FIG. 2. The first surface 170a of the light source 170 disposed in each of the second light source placement portions 16 faces the second window 17. The ultraviolet light emitted from the first surface 170a is emitted via the second window 17 to the fluid flowing in the branch flow channels 80a and 80b.

The light source 170 is disposed in each of the second light source placement portions 16 with the spring members 176 being elastically deformed from a natural state. The spring members 176 disposed on the first surface 170a side contact the second window 17. The restoring force of the spring members 176 causes the light source 170 to be preloaded toward a second partition wall 16b that partitions the downstream flow channel 110 and each of the second light source placement portions 16, and the second surface 170b is pressed against the second partition wall 16b. Accordingly, the cooling efficiency of the light source 170 by the fluid flowing in the downstream flow channel 110 can be increased.

The first region 181 of the light source 170 disposed in the second light source placement portion 16 facing the branch flow channel 80a of the pair of branch flow channels 80a and 80b faces the first flow channel 81a, and light emitting elements disposed in the first region 181 emit ultraviolet light to the fluid flowing in the first flow channel 81a. The second region 182 of the light source 170 disposed in the second light source placement 16 facing the branch flow channel 80a faces the second flow channel 82a, and light emitting elements disposed in the second region 182 emit ultraviolet light to the fluid flowing in the second flow channel 82a.

The first region 181 of the light source 170 disposed in the second light source placement portion 16 facing the branch flow channel 80b faces the second flow channel 82b, and light emitting elements disposed in the first region 181 emit ultraviolet light to the fluid flowing in the second flow channel 82b. The second region 182 of the light source 170 disposed in the second light source placement 16 facing the branch flow channel 80b faces the first flow channel 81b, and light emitting elements disposed in the second region 182 emit ultraviolet light to the fluid flowing in the first flow channel 81b. The ultraviolet light from the light emitting elements can be emitted in the extending direction of each of the first flow channels 81a and 81b and the second flow channels 82a and 82b, and thus the integrated luminance can be increased. The same light emitting elements can be used as the light emitting elements disposed in the first region 181 and the light emitting elements disposed in the second region 182. Light emitting elements having different emission peak wavelengths may be used as the light emitting elements disposed in the first region 181 and the light emitting elements disposed in the second region 182.

The third region 183 of the light source 170 disposed in the second light source placement portion 16 facing the branch flow channel 80a faces the first partition member 61 through the portion where the first flow channel 81a communicates with the second flow channel 82a in the branch flow channel 80a. No light emitting element is disposed in the third region 183 facing the first partition member 61. The third region 183 of the light source 170 disposed in the second light source placement portion 16 facing the branch flow channel 80b faces the fourth partition member 64 through the portion where the first flow channel 81b communicates with the second flow channel 82b in the branch flow channel 80b. No light emitting element is disposed in the third region 183 facing the fourth partition member 64. The integrated luminance of ultraviolet light, emitted from the light emitting elements disposed in the first region 181 and in the second region 182 to the fluid flowing in the branch flow channels 80a and 80b, can be sufficiently obtained. Therefore, the configuration in which no light emitting element is disposed in the third region 183 allows the number of light emitting elements to be reduced while ensuring the effect of treating the fluid with the ultraviolet light.

In the light source 170, a screw 177 illustrated in FIG. 4A can be disposed in the third region 183 where no light emitting element is disposed. The third region 183 including the center of the wiring substrate 171 can be fixed to the holding member 173 by the screw 177. In addition, for example, the four corners of the wiring substrate 171 are fixed to the holding member 173 by screws. By fixing the third region 183 including the center of the wiring substrate 171 to the holding member 173 by the screw 177, a center portion of the wiring substrate 171 can be prevented from being loosened from the holding member 173, and thus, the wiring substrate 171 can be firmly attached to the holding member 173. As a result, the occurrence of a gap between the wiring substrate 171 and the first partition wall 13b can be minimized, and the cooling efficiency of the light source 170 by the fluid flowing through the upstream flow channel 12 can be increased. Further, the occurrence of a gap between the wiring substrate 171 and the second partition wall 16b can be minimized, and the cooling efficiency of the light source 170 by the fluid flowing through the downstream flow channel 110 can be increased.

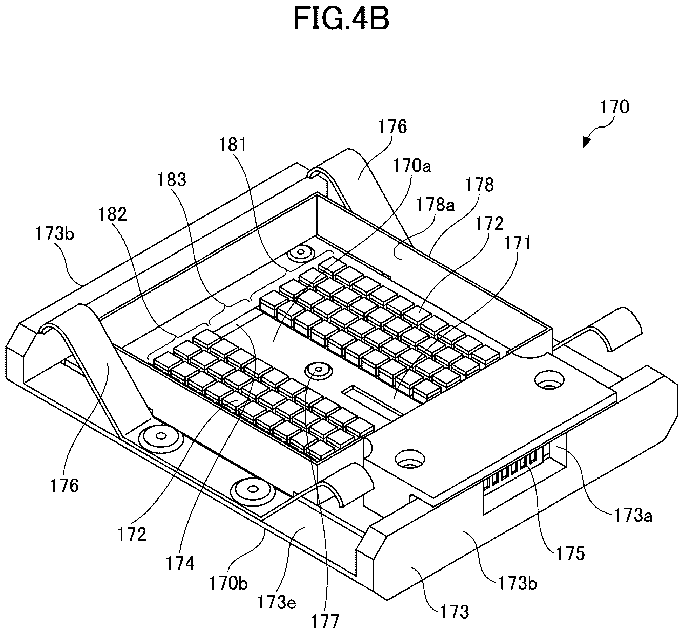

Further, the light source 170 may include a light-reflecting member. FIG. 4B is a perspective view illustrating another example of a light source according to an embodiment of the present invention. In FIG. 4B, a light source 170 including a light-reflecting member 178 is depicted.

The light-reflecting member 178 has, for example, a shape such as a polygonal shape or a circular shape in a plan view as viewed from the surface side of the wiring substrate 171. In the example illustrated in FIG. 4B, the light-reflecting member 178 has a substantially rectangular frame shape in a plan view as viewed from the surface side of the wiring substrate 171. Further, the light-reflecting member 178 is a member having a predetermined height from the surface of the wiring substrate 171. The light-reflecting member 178 surrounds the first region 181, the second region 182, and the third region 183 in a plan view as viewed from the surface side of the wiring substrate 171.

The light-reflecting member 178 includes for example, a metal material, a resin material, or the like. As the metal material, a material whose surface is treated with aluminum, stainless steel, or the like can be used. As the resin material, a fluororesin or the like can be used.

An inner surface 178a of the light-reflecting member 178 reflects light from the light emitting elements included in the first region 181 and the second region 182 to the inside of the light-reflecting member 178. In this manner, of the light from the light-emitting elements, the amount of light emitted to the outside of the light-reflecting member 178 can be reduced. Accordingly, the light extraction efficiency of the light source 170 can be improved.

The inner surface 178a of the light-reflecting member 178 is preferably a surface having high reflectivity with respect to the ultraviolet light emitted from the light emitting elements in order to suppress light loss due to light absorption, light scattering, or the like. Such a surface can be, for example, a surface having a reflectivity of 60% or greater and preferably 90% or greater with respect to the ultraviolet light emitted from the light emitting elements. Instead of the light-reflecting member 178, a member having light absorbency may be disposed.

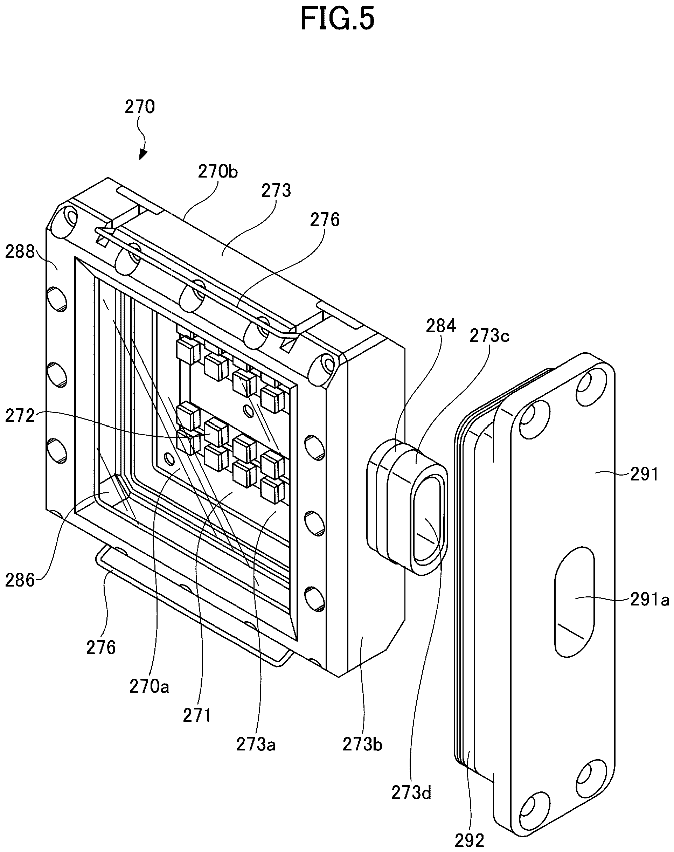

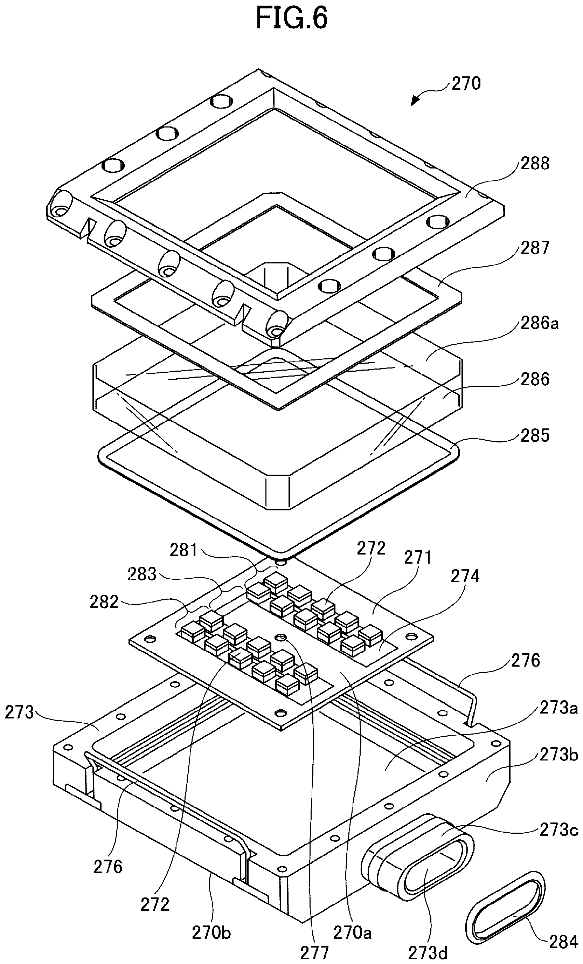

A light source 270 illustrated in FIG. 5 and FIG. 6 can be used as each of the first light source and the second light sources described above. The light source 270 itself has a waterproof structure that shields light emitting elements and a wiring substrate from water. In the following, the description of the same configuration as the configuration of the light source 170 will be omitted as appropriate.

In the light source 270 illustrated in FIG. 5 and FIG. 6, a recess 273a is defined by the surface of a holding member 273 and a wall portion 273b. A wiring substrate 271 is disposed in the recess 273a of the holding member 273.

An opening of the recess 273a of the holding member 273 is closed by a cover glass 286 made of synthetic quartz, for example. A waterproof ring 285 is interposed between the cover glass 286 and the holding member 273. The light source 270 may include a frame member 288 disposed on the wall portion 273b of the holding member 273. The outer shape of the frame member 288 can be a rectangular frame shape in a plan view as viewed from a first surface 270a side of the light source 270. The cover glass 286 is interposed between the frame member 288 and the holding member 273. The frame member 288 is fixed to the holding member 273 by, for example, a screw while a peripheral portion of a surface 286a (opposite to a surface facing the wiring substrate 271) of the cover glass 286 is pressed toward the holding member 273. A cushioning material 287 is interposed between the peripheral portion of the surface 286a of the cover glass 286 and the frame member 288. Note that the frame member 288 may be fixed by an adhesive. Further, in the light source 270, the light-reflecting member 178 as illustrated in FIG. 4B may be disposed in the recess 273a.

A cylindrical portion 273c in which a first through hole 273d leading into the recess 273a is formed is provided on one lateral surface of the wall portion 273b defining the recess 273a of the holding member 273.

The light source 270 can be used as the first light source and disposed in the first light source placement portion 13 through the first opening 13a illustrated in FIG. 2. Further, the light source 270 can be used as each of the second light sources and disposed in the corresponding second light source placement portion 16 through the second opening 16a illustrated in FIG. 2. In a state in which the light source 270 is disposed in the first light source placement portion 13, the first opening 13a is covered by a waterproof cap 291 illustrated in FIG. 5. A waterproof ring 292 is interposed between the waterproof cap 291 and the inner wall of the first opening 13a.

The waterproof cap 291 has a second through hole 291a. The opening shape of the second through hole 291a is preferably substantially the same as that of the cylindrical portion 273c as viewed from the opening direction of the first through hole 273d of the light source 270. In a state in which the light source 270 is disposed in the first light source placement portion 13 and the waterproof cap 291 is inserted into the first opening 13a, the cylindrical portion 273c of the holding member 273 fits into the second through hole 291a. A waterproof ring 284 is interposed between the cylindrical portion 273c and the inner wall of the second through hole 291a. Accordingly, the occurrence of a gap between the second through hole 291a and the cylindrical portion 273c can be prevented, and water can be prevented from entering the first light source placement portion 13. In the light source 270, an electrical cable electrically connected to wiring 274 of the wiring substrate 271 can be disposed on the surface of the wiring substrate 171. In this case, the electrical cable can be pulled out to the outside of the light source 270 through the first through hole 273d of the holding member 273 and the second through hole 291a of the waterproof cap 291.

### <Fluid Retention According to Embodiment>

According to an embodiment, a portion of fluid flowing through the inside of the ultraviolet light fluid treatment device 1 or 2 is retained, and thus the integrated luminance of ultraviolet light emitted to the fluid increases in accordance with the retention time. As a result, the effect of treating the fluid with the ultraviolet light can be enhanced. This fluid retention will be described in detail below. In the following description, the ultraviolet light fluid treatment device 1 is exemplified; however, effects of fluid retention can be similarly obtained in any of the ultraviolet light fluid treatment devices 1 and 2.

### (First Example of Fluid Retention)

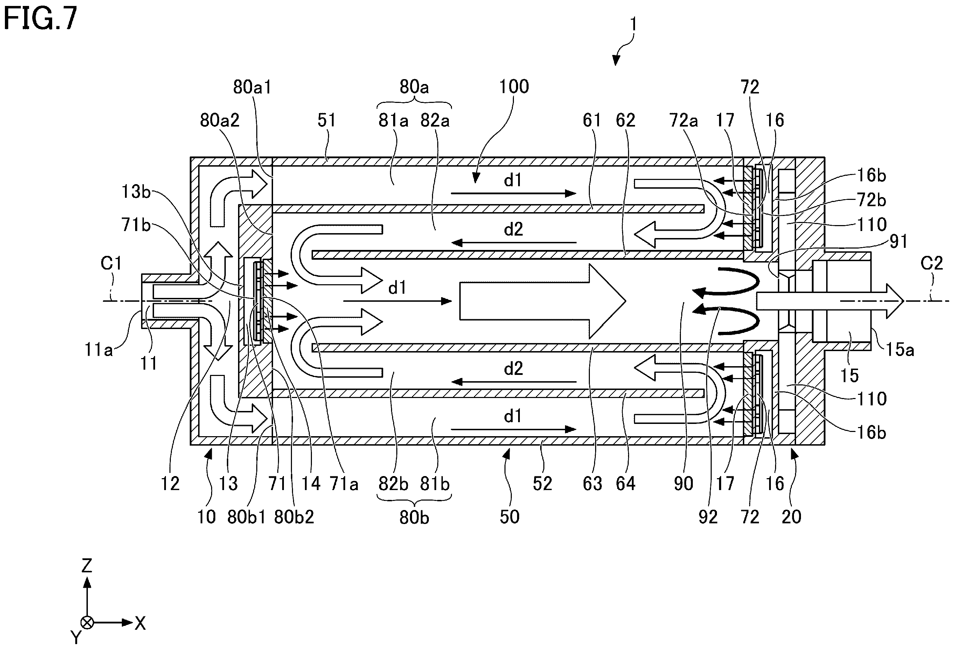

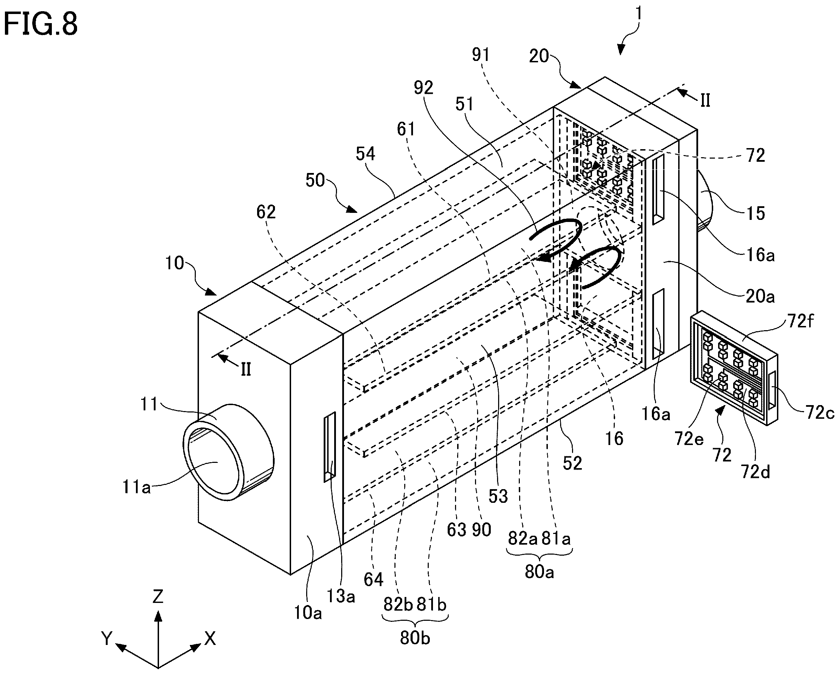

A first example of fluid retention will be described with reference to FIG. 7 and FIG. 8. FIG. 7 and FIG. 8 are drawings illustrating the first example of fluid retention. FIG. 7 is a cross-sectional view of the ultraviolet light fluid treatment device 1. FIG. 8 is a perspective view of the ultraviolet light fluid treatment device 1.

An end portion 91 on the outlet 15 side of the merged flow channel 90 has an opening having a cross-sectional area smaller than the cross-sectional area of the merged flow channel 90 in the direction orthogonal to the first direction d1. As illustrated in FIG. 7 and FIG. 8, a portion of fluid flowing in the merged flow channel 90 from the inlet 11 side toward the outlet 15 side in the X-axis direction is retained by hitting and bouncing back from the end portion 91 on the outlet 15 side of the merged flow channel 90. Flows 92 indicated by bold arrows in FIG. 7 and FIG. 8 represent the flows of the fluid bounced back from the end portion 91.

For example, such flows 92 increase the time during which a portion of the fluid flowing in the merged flow channel 90 is retained in the vicinity of the end portion 91. The flows 92 in FIG. 7 and FIG. 8 are examples illustrated for convenience of description, and the directions and the magnitudes of the flows are not limited thereto.

The first light source 71 is disposed to face the end portion 91, and thus, the fluid retained in the vicinity of the end portion 91 is efficiently irradiated with ultraviolet light from the first light source 71. As a result, the integrated luminance of the ultraviolet light emitted from the first light source 71 to the fluid retained in the vicinity of the end portion 91 increases in accordance with the retention time. Accordingly, the ultraviolet light fluid treatment device 1 can enhance the treatment effect with the ultraviolet light.

### (Second Example of Fluid Retention)

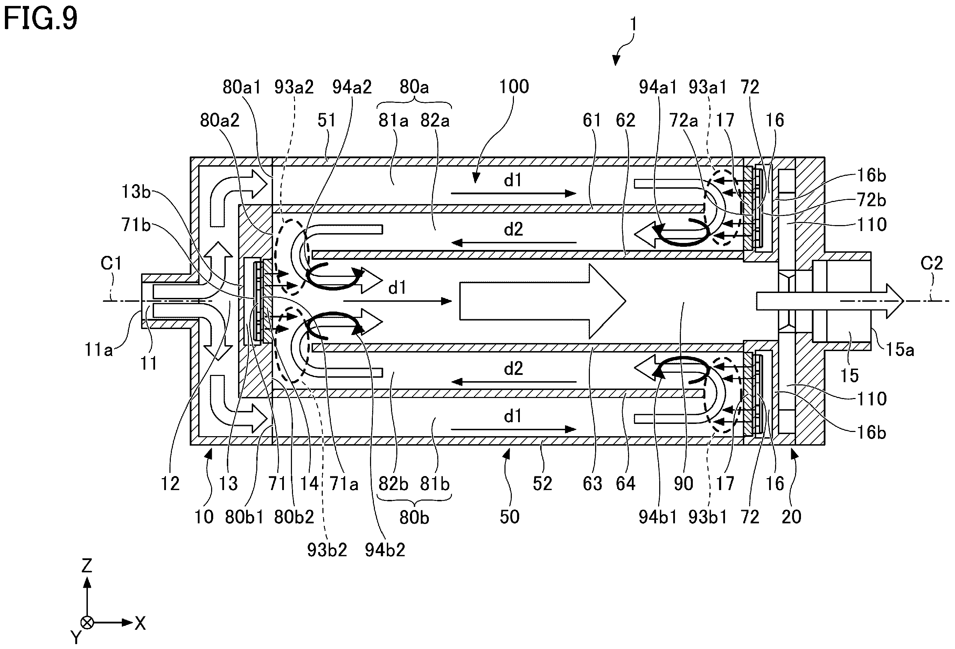

Next, FIG. 9 is a cross-sectional view of the ultraviolet light fluid treatment device 1, which illustrates a second example of fluid retention.

As illustrated in FIG. 9, a portion of fluid flowing in the branch flow channels 80a and 80b in the X-axis direction generates a vortex in turning back portions of the branch flow channels 80a and 80b. As used herein, the term "vortex" refers to a swirling-like flow of fluid.

In FIG. 9, a turning back portion 93a1 indicated by a dashed line is a turning back portion from the first flow channel 81a to the second flow channel 82a in the branch flow channel 80a. A vortex 94a1 indicated by a bold arrow represents a vortex generated in the vicinity of the turning back portion 93a1 in the second flow channel 82a. The turning back portion refers to a portion in which the fluid flowing in a predetermined direction turns back in a direction opposite to the predetermined direction.

Similarly, a turning back portion 93a2 is a turning back portion from the second flow channel 82a in the branch flow channel 80a to the merged flow channel 90. A vortex 94a2 represents a vortex generated in the vicinity of the turning back portion 93a2 in the merged flow channel 90.

A turning back portion 93b1 is a turning back portion from the first flow channel 81b to the second flow channel 82b in the branch flow channel 80b. A vortex 94b1 represents a vortex generated in the vicinity of the turning back portion 93b1 in the second flow channel 82b.

A turning back portion 93b2 is a turning back portion from the second flow channel 82b to the merged flow channel 90 in the branch flow channel 80b. A vortex 94b2 represents a vortex generated in the vicinity of the turning back portion 93b2 in the merged flow channel 90.

In particular, the cross-sectional shape of the ultraviolet light fluid treatment device 1 orthogonal to the direction in which the fluid flows is a substantially rectangular shape. Thus, vortices are likely to occur at the turning back portions 93a1, 93a2, 93b1, and 93b2 near corner portions. As used herein, the term "corner portion" refers to a portion where surfaces meet.

The generation of vortices such as the vortices 94a1, 94a2, 94b1, and 94b2 increases the time during which a portion of the fluid flowing in the branch flow channels 80a and 80b is retained in the vicinity of each of the turning back portions 93a1, 93a2, 93b1, 93b2. The vortices 94a1, 94a2, 94b1, and 94b2 in FIG. 9 are examples illustrated for convenience of description, and the directions and the magnitudes of the vortices are not limited thereto.

The first light source 71 is disposed in the vicinity of each of the turning back portions 93a2 and 93b2, and thus the fluid retained in the vicinity of each of the turning back portions 93a2 and 93b2 is efficiently irradiated with the ultraviolet light from the first light source 71. Further, the second light sources 72 are disposed in the vicinities of the turning back portions 93a1 and 93b1, and thus the fluid retained in the vicinities of the turning back portions 93a1 and 93b1 is efficiently irradiated with the ultraviolet light from the second light sources 72.

As described above, the integrated luminance of the ultraviolet light from the first light source 71 and the second light sources 72 to the fluid retained in the vicinities of the turning back portions 93a1, 93a2, 93b1, and 93b2 increases in accordance with the retention time. Accordingly, the ultraviolet light fluid treatment device 1 can enhance the effect of treating the fluid with the ultraviolet light.

Further, if the fluid is, for example, water, because bacteria and viruses in the water have a high specific gravity relative to the water, the bacteria and viruses are likely to pass near the first light source 71 and the second light sources 72 by centrifugal force when flowing through the turning back portions 93a1, 93a2, 93b1, and 93b2. Therefore, the ultraviolet light fluid treatment device 1 can enhance the effect of treating the bacteria and viruses in the water with the ultraviolet light.

In the example illustrated in FIG. 9, the ultraviolet light fluid treatment device 1 including the plurality of branch flow channels 80a and 80b is exemplified; however, the configuration is not limited thereto. Even when the ultraviolet light fluid treatment device 1 includes one branch flow channel, the effects described in the second example can be obtained.

### (Third Example of Fluid Retention)

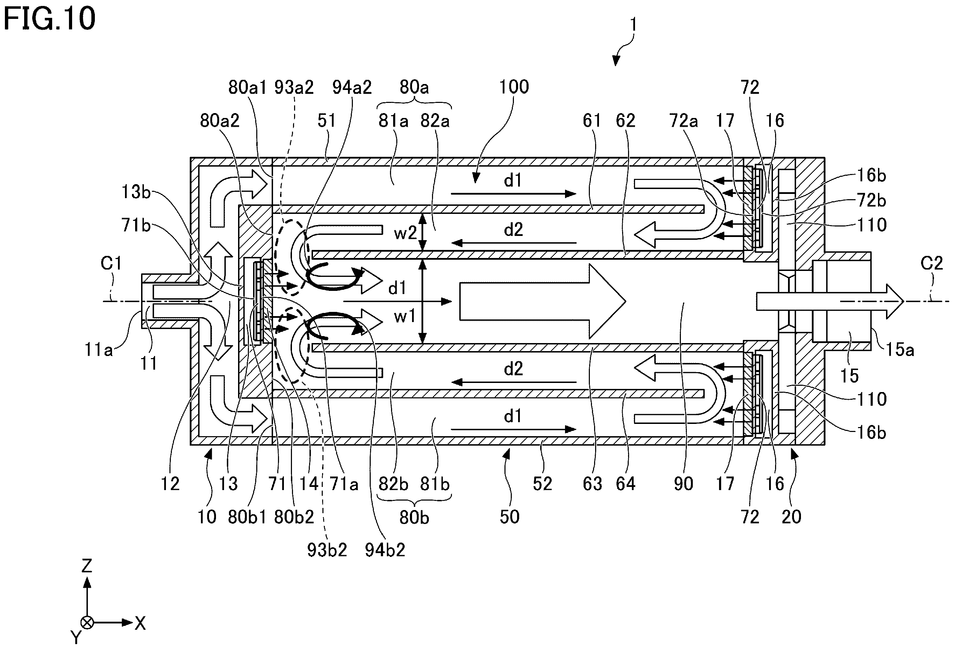

Next, FIG. 10 is a cross-sectional view of the ultraviolet light fluid treatment device 1, which illustrates a third example of fluid retention.

As illustrated in FIG. 10, by causing a width w1 of the merged flow channel 90 to be greater than a width w2 of each of the branch flow channels 80a and 80b in a direction orthogonal to the direction in which the fluid flows, a vortex is likely to be generated. The direction orthogonal to the direction in which the fluid flows is, for example, a direction along the Y-axis or a direction along the Z-axis. Accordingly, in the ultraviolet light fluid treatment device 1, the width w1 along the Y-axis may be greater than the width w2 along the Y-axis, or the width w1 along the Z-axis may be greater than the width w2 along the Z-axis.

By causing the width w1 to be greater than the width w2, a flow rate difference or a flow velocity difference occurs between the fluid flowing in each of the branch flow channels 80a and 80b and the fluid flowing in the merged flow channel 90. Because of this flow rate difference or flow velocity difference, a vortex is more likely to be generated in the vicinity of each of the turning back portions 93a2 and 93b2, thereby increasing the retention time of the fluid in the vicinity of each of the turning back portions 93a2 and 93b2. In FIG. 10, the vortex 94a2 represents a vortex generated in the vicinity of the turning back portion 93a2 in the merged flow channel 90, and the vortex 94b2 represents a vortex generated in the vicinity of the turning back portion 93b2 in the merged flow channel 90.

Effects of fluid retention in the vicinity of each of the turning back portions 93a2 and 93b2 are similar to those described in the above second example.

### (Fourth Example of Fluid Retention)

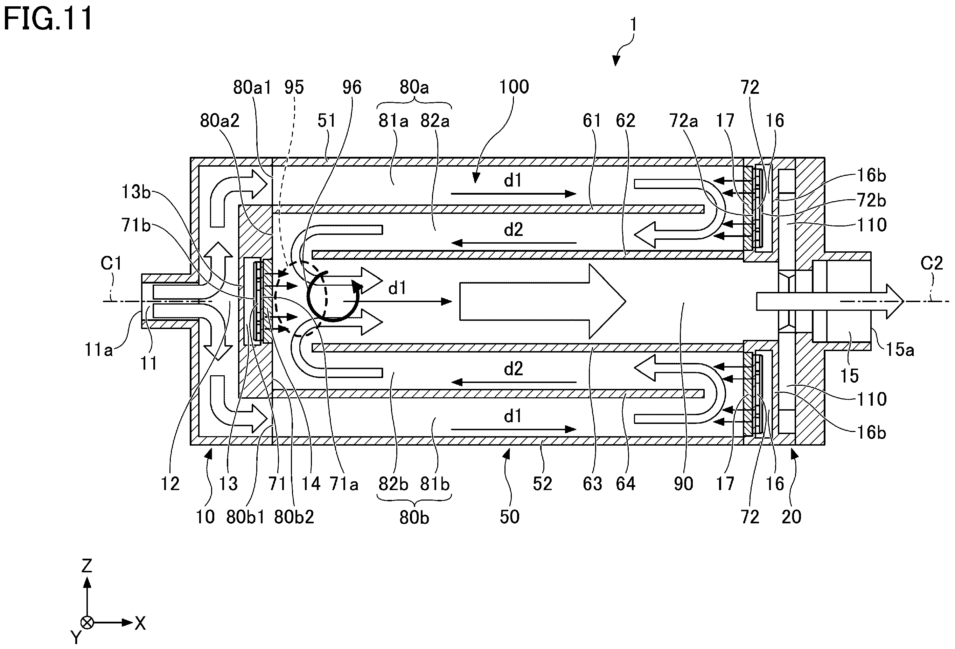

Next, FIG. 11 is a cross-sectional view of the ultraviolet light fluid treatment device 1, which illustrates a fourth example of fluid retention.

A illustrated in FIG. 11, when the ultraviolet light fluid treatment device 1 is disposed such that the branch flow channel 80b is positioned vertically below the branch flow channel 80a, a vortex is likely to be generated in the vicinity of a merging portion 95 located on the inlet 11 side of the merged flow channel 90. In FIG. 11, the Z-axis is in the vertical direction.

By positioning the branch flow channel 80b vertically below the branch flow channel 80a, a flow rate difference or a flow velocity difference occurs between the fluid flowing from the branch flow channel 80a into the merged flow channel 90 and the fluid flowing from the branch flow channel 80b into the merged flow channel 90 by the action of gravity. For example, the flow rate of the fluid flowing from the branch flow channel 80a into the merged flow channel 90 increases relative to the flow rate of the fluid flowing from the branch flow channel 80b into the merged flow channel 90 by the action of gravity. Because of this flow rate difference or flow velocity difference, a vortex is more likely to be generated in the vicinity of the merging portion 95 where the fluid merges, thereby increasing the retention time of the fluid in the vicinity of the merging portion 95.

In FIG. 11, a vortex 96 represents a vortex generated in the vicinity of the merging portion 95. The vortex 96 in FIG. 11 is an example illustrated for convenience of description, and the direction and the magnitude of the vortex are not limited thereto.

The first light source 71 is disposed in the vicinity of the merging portion 95, and thus the fluid retained in the vicinity of the merging portion 95 is efficiently irradiated with the ultraviolet light from the first light source 71. As a result, the integrated luminance of the ultraviolet light irradiated from the first light source 71 to the fluid retained in the vicinity of the merging portion 95 increases in accordance with the retention time. Accordingly, the ultraviolet light fluid treatment device 1 can enhance the treatment effect with the ultraviolet light.

### (Fifth Example of Fluid Retention)

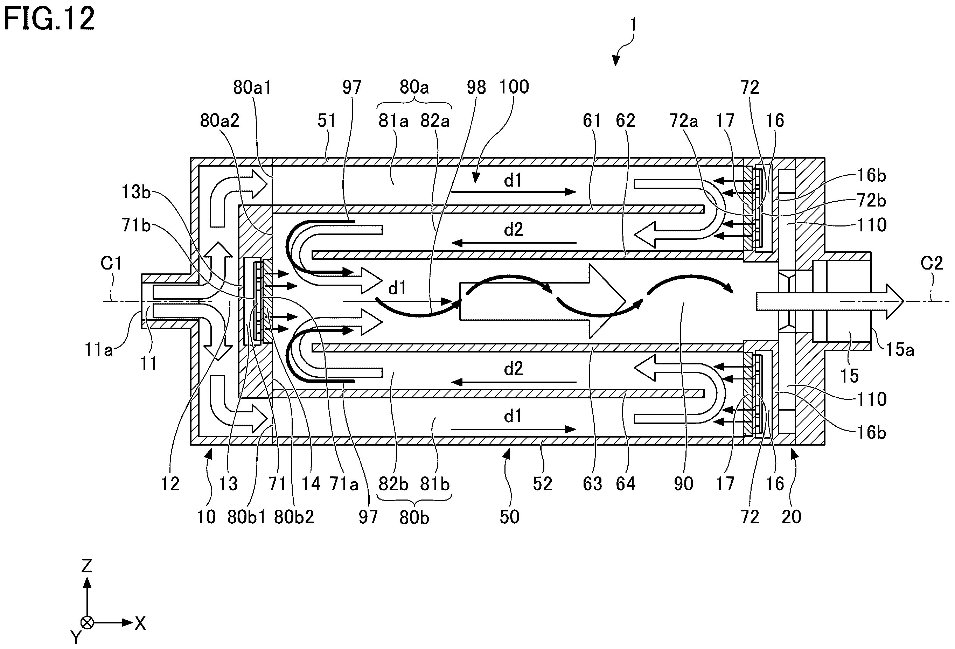

Next, FIG. 12 is a cross-sectional view of the ultraviolet light fluid treatment device 1, which illustrates a fifth example of fluid retention.

Similarly to the fourth example described above, in the fifth example as well, by positioning the branch flow channel 80b vertically below the branch flow channel 80a, a flow rate difference or a flow velocity difference occurs between the fluid flowing from the branch flow channel 80a into the merged flow channel 90 and the fluid flowing from the branch flow channel 80b into the merged flow channel 90. This flow rate difference or flow velocity difference causes the fluid to flow in a meandering manner in the merged flow channel 90 as illustrated in FIG. 12.

In FIG. 12, flows 97 represent the flows of the fluid flowing from the branch flow channels 80a and 80b into the merged flow channel 90, and a flow 98 represents the flow of the fluid flowing in the merged flow channel 90 in a meandering manner. For example, the flow rate of the fluid flowing from the branch flow channel 80a into the merged flow channel 90 increases relative to the flow rate of the fluid flowing from the branch flow channel 80b into the merged flow channel 90 by the action of gravity. Thus, immediately after the fluid merges, the fluid tends to flow vertically downward in the merged flow channel 90. In other words, immediately after the fluid merges in the merged flow channel 90, a vector in the flow direction of the fluid from the inlet 11 toward the outlet 15 tends to be tilted with respect to the X-axis toward a branch flow channel having a lower flow rate of the branch flow channels 80a and 80b. In this example, the branch flow channel having a lower flow rate of the branch flow channels 80a and 80b is on the vertically downward side.

The fluid flowing in the merged flow channel 90 with the vector in the flow direction being tilted vertically downward is bounced back vertically upward by the third partition member 63, then flows in the merged flow channel 90 with the vector in the flow direction being tilted vertically upward, and is subsequently bounced back vertically downward by the second partition member 62. By repeating such movement, the fluid flows in the merged flow channel 90 in a meandering manner.

When the fluid flows in the merged flow channel 90 in a meandering manner, the distance in which the fluid flows in the merged flow channel 90 increases. Therefore, it is conceivable that the time during which the fluid is retained in the merged flow channel 90 increases. The flows 97 and 98 in FIG. 12 are examples illustrated for convenience of description, and the directions and the magnitudes of the flows are not limited thereto.

The meandering flow such as the flow 98 of the fluid is a phenomenon that is more pronounced when the branch flow channels 80a and 80b are separated from each other by the second partition member 62, the third partition member, and the like. In other words, by including the branch flow channels 80a and 80b separated from each other in the ultraviolet light fluid treatment device 1, a flow rate difference or a flow velocity difference occurs between the fluid flowing in the branch flow channel 80a and the fluid flowing in the branch flow channel 80b, thereby allowing the fluid to flow in a meandering manner as in the flow 98.

For example, if a plurality of flow channels each has an annular cross-sectional shape orthogonal to a direction in which fluid flows, a multi-tube structure can be configured in which the flow channels are formed concentrically. However, in the case of such a multi-tube structure, because the flow channels are connected in a circumferential direction, a flow rate difference or a flow velocity difference is less likely to occur in the fluid flowing in the flow channels. Therefore, in a configuration in which each of the flow channels has an annular shape, it would be difficult for the fluid to flow in a meandering manner as in the flow 98.

The first light source 71 is disposed such that the ultraviolet light from the first light source 71 is efficiently emitted to the fluid flowing in the merged flow channel 90. By causing the fluid to flow in a meandering manner in the merged flow channel 90, the retention time increases, thereby increasing the integrated luminance. Accordingly, the ultraviolet light fluid treatment device 1 can enhance the treatment effect with the ultraviolet light.

Further, if the fluid is, for example, water, it is conceivable that because bacteria and viruses in the water have a high specific gravity relative to the water, the retention time increases by centrifugal force when the fluid flows in a meandering manner in the merged flow channel 90. Therefore, the ultraviolet light fluid treatment device 1 can increase the integrated luminance of the ultraviolet light emitted to the bacteria and viruses in the water, and can enhance the treatment effect with the ultraviolet light.

### (Sixth Example of Fluid Retention)

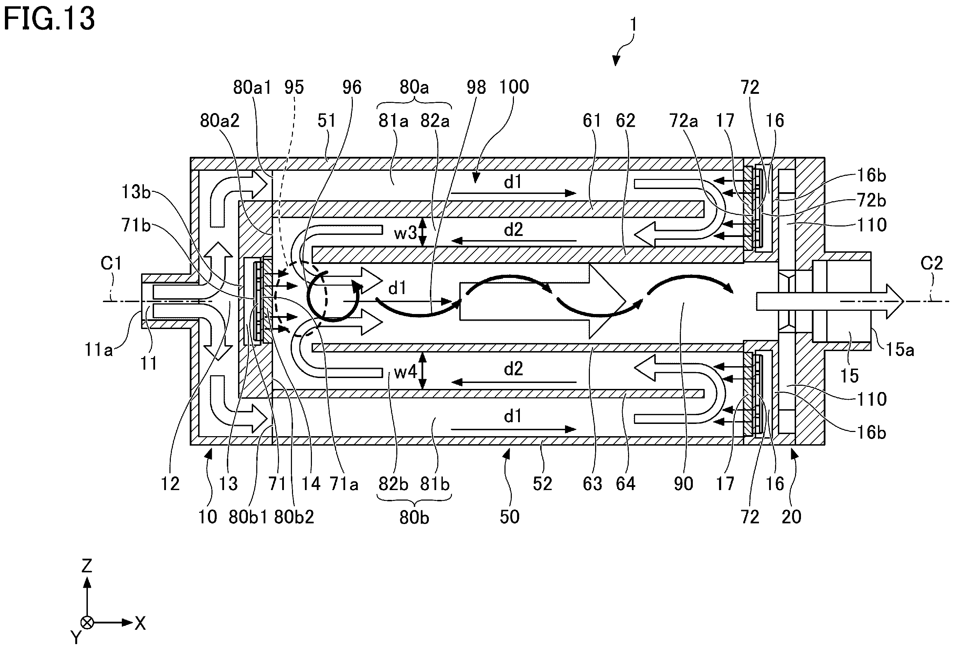

Next, FIG. 13 is a cross-sectional view of the ultraviolet light fluid treatment device 1, which illustrates a sixth example of fluid retention.

In the sixth example, the width of at least one of the plurality of branch flow channels in a direction orthogonal to the direction in which the fluid flows differs from the width of any other branch flow channel of the plurality of branch flow channels in the direction orthogonal to the direction in which the fluid flows. In the example illustrated in FIG. 13, a width w3 of the branch flow channel 80a differs from a width w4 of the branch flow channel 80b in the direction orthogonal to the direction in which the fluid flows. When the width w3 of the branch flow channel 80a differs from the width w4 of the branch flow channel 80b in the direction orthogonal to the direction in which the fluid flows, vortices and meandering are more likely to occur.

The direction orthogonal to the direction in which the fluid flows is, for example, a direction along the Y-axis or a direction along the Z-axis. Accordingly, in the ultraviolet light fluid treatment device 1, the width w3 along the Y-axis and the width w4 along the Y-axis may differ, or the width w3 along the Z-axis and the width w4 along the Z-axis may differ. In the sixth example, the installation orientation of the ultraviolet light fluid treatment device 1 is not particularly limited. This is also true for the other examples of fluid retention, except for the fourth example and the fifth example.

FIG. 13 illustrates a configuration in which the width w3 of the second flow channel 82a in branch flow channel 80a is smaller than the width w4 of the second flow channel 82b in the branch flow channel 80b, thereby making the widths different from each other; however, the configuration is not limited thereto. For example, the width w3 of the second flow channel 82a may be greater than the width w4 of the second flow channel 82b, thereby making the widths different from each other. Further, the width of the first flow channel 81a in the branch flow channel 80a may differ from the width of the first flow channel 81b in the branch flow channel 80b in the direction orthogonal to the direction in which the fluid flows. In FIG. 13, the width of the first flow channel 81a and the width of the second flow channel 82a are the same in the direction orthogonal to the direction in which the fluid flows, but may differ from each other. Further, the width of the first flow channel 81b and the width of the second flow channel 82b are the same, but may differ from each other.

By causing the width w3 and the width w4 to differ from each other, a flow rate difference or a flow velocity difference occurs between the fluid flowing from the branch flow channel 80a into the merged flow channel 90 and the fluid flowing from the branch flow channel 80b into the merged flow channel 90. For example, by causing the width w3 to be smaller than the width w4, the flow rate of the fluid flowing from the branch flow channel 80a into the merged flow channel 90 decreases as compared to the flow rate of the fluid flowing from the branch flow channel 80b into the merged flow channel 90. Because of this flow rate difference or flow velocity difference, a vortex is more likely to occur in the vicinity of the merging portion 95 where the fluid merges, thereby increasing the retention time of the fluid in the vicinity of the merging portion 95. Further, because of the flow rate difference or the flow velocity difference, the fluid flows in a meandering manner in the merged flow channel 90, thereby increasing the retention time of the fluid in the merged flow channel 90.

Effects resulting from a vortex such as the vortex 96 in the merging portion 95 are similar to those described in the above fourth example. Further, effects resulting from a meandering flow such as the flow 98 of the fluid in the merged flow channel 90 are similar to those described in the above fifth example.

In the above embodiments, the two branch flow channels 80a and 80b are exemplified as the plurality of branch flow channels; however the configuration is not limited thereto. If the ultraviolet light fluid treatment device 1 includes three or more branch flow channels, the effects described in the sixth example can be obtained as long as the width of at least one of the three or more branch flow channels in the direction orthogonal to the direction in which the fluid flows differs from the width of any other branch flow channel in the direction orthogonal to the direction in which the fluid flows.

### (Seventh Example of Fluid Retention)

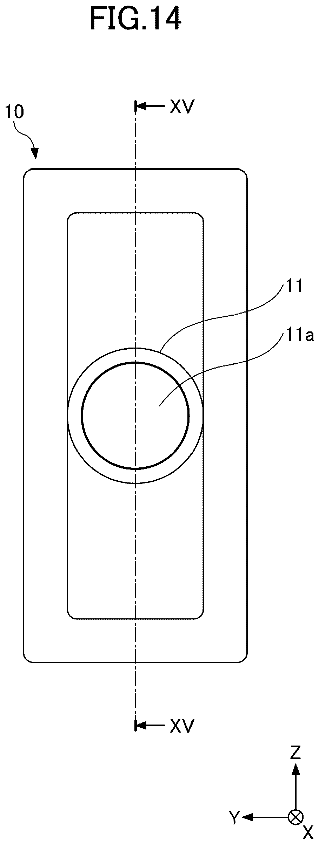

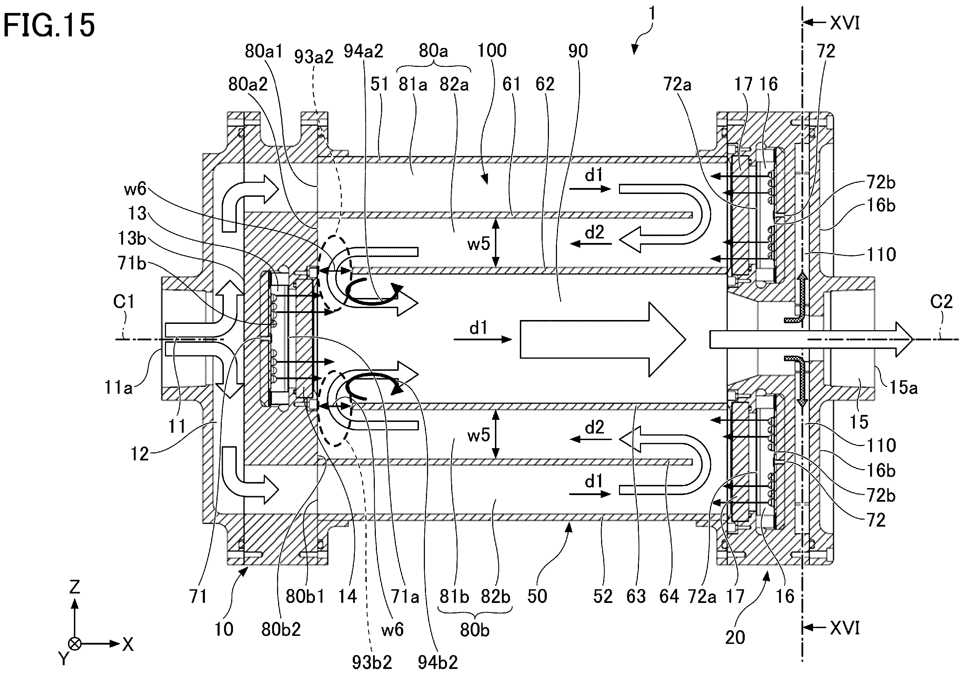

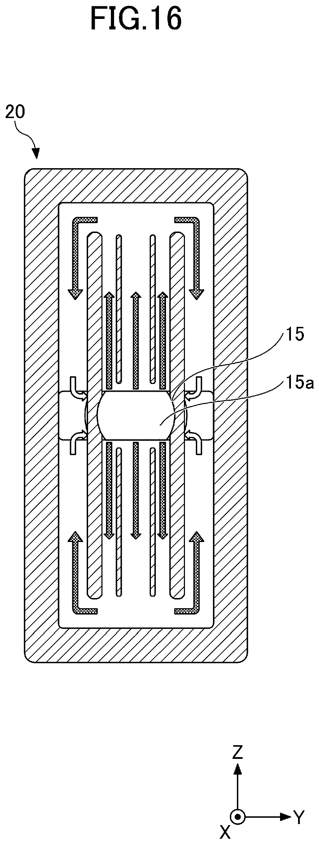

Next, FIG. 14 to FIG. 16 are drawings illustrating the ultraviolet light fluid treatment device 1, which illustrates a seventh example of fluid retention. FIG. 14 is a side view of the ultraviolet light fluid treatment device 1 as viewed from the inlet 11 side. FIG. 15 is a cross-sectional view of the ultraviolet light fluid treatment device 1 taken through XV-XV of FIG. 14. FIG. 16 is a cross-sectional view of the ultraviolet light fluid treatment device 1 taken through XVI-XVI of FIG. 15, as viewed from the outlet 15 side.

In FIG. 14 to FIG. 16, white arrows indicate flows, serving as primary flows, of portions of fluid flowing into the ultraviolet light fluid treatment device 1 through the inlet 11 and flowing out of the ultraviolet light fluid treatment device 1 through the outlet 15. Further, in FIG. 14 to FIG. 16, dot-hatched arrows indicate flows of portions of the fluid used to cool the second light sources 72, of the fluid flowing through the ultraviolet light fluid treatment device 1.

As illustrated in FIG. 15, a vortex is more likely to occur when a width w5 of the branch flow channel 80a in a direction orthogonal to the extending direction of the branch flow channels 80a and 80b differs from a width w6 of the turning back portions 93a2 and 93b2 in a direction along the extending direction of the branch flow channels 80a and 80b.

The extending direction of the branch flow channels 80a and 80b is, for example, a direction along the X-axis. The direction orthogonal to the extending direction of the branch flow channels 80a and 80b is, for example, a direction along the Y-axis or a direction along the Z-axis. Therefore, in the ultraviolet light fluid treatment device 1, the width w5 along the Y-axis and the width w6 along the X-axis may differ from each other, or the width w5 along the Z-axis and the width w6 along the X-axis may differ from each other.

By causing the width w5 and the width w6 to differ from each other, a flow rate difference or a flow velocity difference occurs between the fluid flowing in the branch flow channels 80a and 80b and the fluid flowing in the turning back portions 93a2 and 93b2. Because of this flow rate difference or flow velocity difference, a vortex is more likely to occur in the vicinity of each of the turning back portions 93a2 and 93b2, thereby increasing the retention time of the fluid in the vicinity of each of the turning back portions 93a2 and 93b2.

Effects of fluid retention in the vicinity of each of the turning back portions 93a2 and 93b2 are similar to those described in the above second example. Further, similar effects of fluid retention can be obtained in the turning back portions 93a1 and 93a2 as well.

In the ultraviolet light fluid treatment device 1 illustrated in FIG. 15, the length of each of the first flow channels 81a and 81b and the second flow channels 82a and 82b in the X-axis direction is 200 mm, for example. Further, the width of each of the first flow channels 81a and 81b and the second flow channels 82a and 82b in the Z-axis direction is, for example, 24 mm. The width of each of the first flow channels 81a and 81b and the second flow channels 82a and 82b in the Y-axis direction is, for example, 50 mm. The flow rate of the fluid flowing through the ultraviolet light fluid treatment device 1 illustrated in FIG. 15 is preferably 3 m³/h or more. By ensuring a flow rate of 3 m³/h or more, air bubbles remaining in the ultraviolet light fluid treatment device 1 can be reduced. Accordingly, a reduction in integrated luminance due to the ultraviolet light, emitted from the first light source 71 and the second light sources 72, being reflected or scattered by air bubbles can be suppressed. As a result, the ultraviolet light fluid treatment device 1 can increase the integrated luminance of the ultraviolet light, and can enhance the treatment effect with the ultraviolet light. Further, in the ultraviolet light fluid treatment device 1, a decrease in heat transfer efficiency due to air bubbles entering the downstream flow channel 110 can be suppressed, and a decrease in cooling efficiency of the second light sources 72 due to the fluid flowing in the downstream flow channel 110 can be suppressed. The dimensions of the ultraviolet light fluid treatment device 1 and the flow rate of the fluid are not limited to the above.

### [Third Embodiment]

An ultraviolet light fluid treatment device according to a third embodiment will be described. The same components as those of the first and second embodiments are denoted by the same reference numerals, and a duplicate description will be omitted as appropriate. This applies to other embodiments described later.

If the ultraviolet light fluid treatment device is not used for a certain period of time while liquid is contained inside the device, mold and bacteria may grow inside the device. Such mold and bacteria would decrease the treatment effect of the ultraviolet light fluid treatment device. Therefore, in order to prevent the growth of mold and bacteria, the liquid inside the ultraviolet light fluid treatment device is preferably drained periodically or before a period of non-usage, such that no liquid remains in the device, that is, the ultraviolet light fluid treatment device is emptied.

In the present embodiment, the ultraviolet light fluid treatment device includes a drain port on the lower side in the vertical direction. The drain port is configured to discharge liquid inside the device to the outside. Further, in the ultraviolet light fluid treatment device according to the present embodiment, partition members that partition flow channels of the liquid are inclined with respect to the horizontal direction orthogonal to the vertical direction. With this configuration, when the liquid inside the device is discharged to empty the device, the liquid can easily flow vertically downward and can be easily discharged through the drain port to the outside. Accordingly, residual liquid in the device can be reduced.

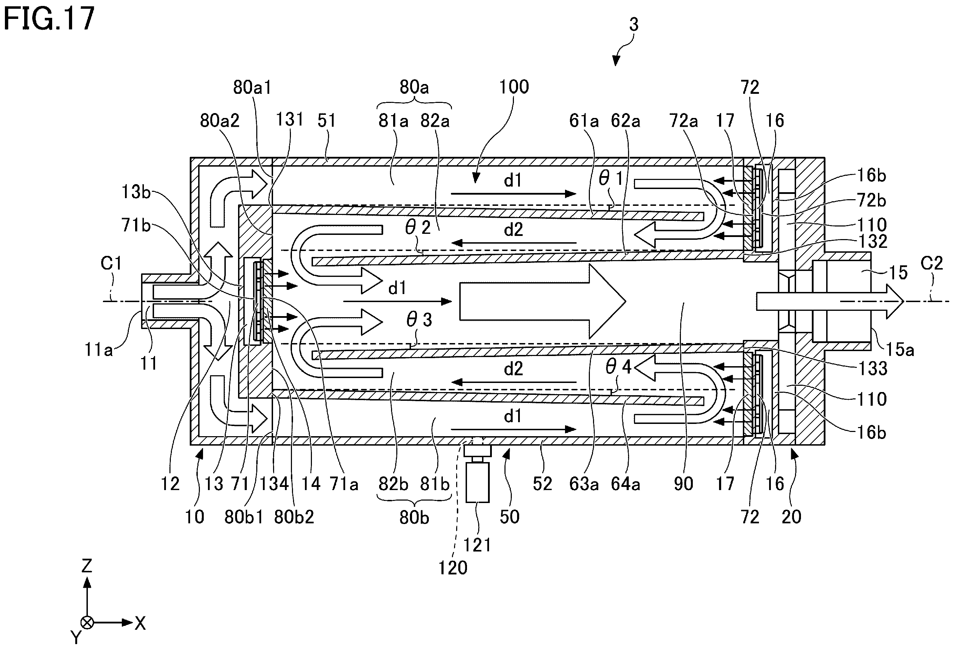

FIG. 17 is a cross-sectional view of an ultraviolet light fluid treatment device 3 according to the third embodiment of the present invention. As illustrated in FIG. 17, the ultraviolet light fluid treatment device 3 includes a drain port 120, a drain mechanism 121, and a plurality of partition members 61a to 64a. As the plurality of partition members 61a to 64a, for example, four partition members (a first partition member 61a, a second partition member 62a, a third partition member 63a, and a fourth partition member 64a) are disposed in the intermediate portion 50.

The drain port 120 is a through hole formed in the second wall 52 located on the lower side in the vertical direction of the intermediate portion 50 in the ultraviolet light fluid treatment device 3. The drain port 120 can be opened and closed by a plug. In a state in which the plug of the drain port 120 is opened, the liquid inside the ultraviolet light fluid treatment device 3 flows vertically downward by the action of gravity and is discharged through the drain port 120. In a state in which the plug of the drain port 120 is closed, the liquid inside the ultraviolet light fluid treatment device 3 is not discharged. The position where the drain port 120 is formed is not particularly limited as long as the drain port 120 is formed in the second wall 52.

The drain mechanism 121 is a mechanism that can adjust the amount of liquid discharged from the drain port 120. For example, the drain mechanism 121 is a drain device including a plug that can adjust opening and closing of the drain port 120. The amount of liquid discharged from the drain port 120 can be adjusted by adjusting opening and closing of the plug of the drain device in the ultraviolet light fluid treatment device 3. The drain mechanism 121 is not an essential component; however, the ultraviolet light fluid treatment device 3 preferably includes the drain mechanism 121 from the viewpoint of improving the workability of a drain operation.

The shape, the arrangement position, and the support method of the first partition member 61a are the same as those of the first partition member 61 according to the first embodiment. The first partition member 61a differs from the first partition member 61 in that the first partition member 61a is inclined at an angle Θ1 with respect to the X-axis direction. The X-axis direction is orthogonal to the Z-axis direction, and corresponds to the horizontal direction orthogonal to the vertical direction. When the first partition member 61a is inclined at the angle Θ1, the height of the surface of the first partition member 61a in the vertical direction decreases along a direction of the arrow of the X-axis.

The shape, the arrangement position, and the support method of the second partition member 62a are the same as those of the second partition member 62 according to the first embodiment. The second partition member 62a differs from the second partition member 62 in that the second partition member 62a is inclined at an angle Θ2 with respect to the X-axis direction. In the example illustrated in FIG. 17, when the second partition member 62a is inclined at the angle Θ2, the height of the surface of the second partition member 62a in the vertical direction decreases along a direction opposite to the direction of the arrow of the X-axis.

The shape, the arrangement position, and the support method of the third partition member 63a are the same as those of the third partition member 63 according to the first embodiment. The third partition member 63a differs from the third partition member 63 in that the third partition member 63a is inclined at an angle Θ3 with respect to the X-axis direction. When the third partition member 63a is inclined at the angle Θ3, the height of the surface of the third partition member 63a in the vertical direction decreases along the direction opposite to the direction of the arrow of the X-axis.

The shape, the arrangement position, and the support method of the fourth partition member 64a are the same as those of the fourth partition member 64 according to the first embodiment. The fourth partition member 64a differs from the fourth partition member 64 in that the fourth partition member 64a is inclined at an angle Θ4 with respect to the X-axis direction. When the fourth partition member 64a is inclined at the angle Θ4, the height of the surface of the fourth partition member 64a in the vertical direction decreases along the direction of the arrow of the X-axis.

The magnitudes of the angle Θ1, the angle Θ2, the angle Θ3, and the angle Θ4 are not particularly limited and can be appropriately selected. Preferably, each of the angle Θ1, the angle Θ2, the angle Θ3, and the angle Θ4 is approximately 1° from the viewpoint of satisfying the drainage requirements in Good Manufacturing Practice (GMP) standards.

In order to discharge the liquid from the inside of the ultraviolet light fluid treatment device 3, the plug of the drain mechanism 121 is opened, and as a result, the liquid in the first flow channel 81b is discharged vertically downward from the drain port 120 of the ultraviolet light fluid treatment device 3 by the action of gravity.

In response to the liquid in the first flow channel 81b being discharged, the liquid in the second flow channel 82b flows toward the first flow channel 81b along the fourth partition member 64a. The height of the surface of the fourth partition member 64a in the vertical direction decreases along the direction of the arrow of the X-axis. For this reason, the liquid in the second flow channel 82b easily flows toward the first flow channel 81b along the fourth partitioning member 81b by the action of gravity, and is less likely to remain on the fourth partitioning member 64a.

In response to the liquid in the second flow channel 82b flowing into the first flow channel 81b, the liquid in the merged flow channel 90 flows toward the second flow channel 82b along the third partition member 63a. The height of the surface of third partition member 63a in the vertical direction decreases along the direction opposite to the direction of the arrow of the X-axis. For this reason, the liquid in the merged flow channel 90 easily flows toward the second flow channel 82b along the third partition member 63a by the action of gravity, and is less likely to remain on the third partition member 63a.

In response to the liquid in the merged flow channel 90 flowing into the second flow channel 82b, the liquid in the second flow channel 82a flows toward the merged flow channel 90 along the second partition member 62a. The height of the surface of second partition member 62a in the vertical direction decreases along the direction opposite to the direction of the arrow of the X-axis. For this reason, the liquid in the second flow channel 82a easily flows toward the merged flow channel 90 along the second partition member 62a by the action of gravity, and is less likely to remain on the second partition member 62a.

In response to the liquid in the second flow channel 82a flowing into the merged flow channel 90, the liquid in the first flow channel 81a flows toward the second flow channel 82a along the first partition member 61a. The height of the surface of first partition member 61a in the vertical direction decreases along the direction of the arrow of the X-axis. For this reason, the liquid in the first flow channel 81a easily flows toward the second flow channel 82a along the first partition member 61a by the action of gravity, and is less likely to remain on the first partition member 61a.

As described above, when the liquid inside the ultraviolet light fluid treatment device 3 is discharged to empty the device, the liquid inside the device can be easily discharged from the drain port 120 to the outside, thereby allowing the liquid to be less likely to remain in the device.

In FIG. 17, a first downstream end 131 is an end of the first partition member 61a on the downstream side in the direction in which the liquid flows. There may be a gap along the X-axis direction between the first downstream end 131 and the surface of the first end portion 10 facing the first downstream end 131. When the liquid inside the device is discharged to empty the device, the liquid easily flows vertically downward through the gap as compared when there is no gap. Thus, the presence of the gap is preferable.

The length of the gap along the X-axis direction between the first downstream end 131 and the surface of the first end portion 10 is not particularly limited, as long as fluid can pass through the gap. However, if the gap is too large, the treatment effect of the ultraviolet light fluid treatment device 3 would be decreased due to the fluid passing through the gap during treatment. Therefore, the length of the gap along the X-axis direction is preferably approximately 1 mm or more and 5 mm or less.

A second downstream end 132 is an end of the second partition member 62a on the downstream side in the direction in which the fluid flows. Similar to the first downstream end 131, there may be a gap along the X-axis direction between the second downstream end 132 and the surface of the second end portion 20 facing the second downstream end 132. Functions and effects of this gap are the same as those of the first downstream end 131.

A third downstream end 133 is an end of the third partition member 63a on the downstream side in the direction in which the fluid flows. Similar to the first downstream end 131, there may be a gap along the X-axis direction between the third downstream end 133 and the surface of the second end portion 20 facing the third downstream end 133. Functions and effects of this gap are the same as those of the first downstream end 131.

A fourth downstream end 134 is an end of the fourth partition member 64a on the downstream side in the direction in which the fluid flows. Similar to the first downstream end 131, there may be a gap along the X-axis direction between the fourth downstream end 134 and the surface of the first end portion 10 facing the fourth downstream end 134. Functions and effects of this gap are the same as those of the first downstream end 131.

### [Fourth Embodiment]

An ultraviolet light fluid treatment device according to a fourth embodiment will be described.

In the ultraviolet light fluid treatment device, if air bubbles are included in liquid flowing in the flow channels of the ultraviolet light fluid treatment device during treatment, the liquid would not be subjected to the treatment by the volume of the air bubbles, and thus the treatment efficiency of the ultraviolet light fluid treatment device would be decreased. Further, in the ultraviolet light fluid treatment device 1 of the first embodiment, if air bubbles are included in liquid flowing in the downstream flow channel 110, the cooling efficiency of the second light sources 72 would be decreased by the volume of the air bubbles. The term "air bubble" refers to gas in the liquid.

The ultraviolet light fluid treatment device according to the present embodiment includes a first exhaust port configured to discharge air bubbles in liquid filled in the device, and a second exhaust port configured to discharge air bubbles in the liquid flowing in the downstream flow channel. The first exhaust port and the second exhaust port are disposed on the upper side in the vertical direction of the intermediate portion 50. In the present embodiment, the ultraviolet light fluid treatment device includes the first exhaust port, and thus air bubbles in the liquid filled in the device can be reduced. Therefore, a decrease in the treatment efficiency of the ultraviolet light fluid treatment device can be reduced. Further, in the present embodiment, the ultraviolet light fluid treatment device includes the second exhaust port, and thus air bubbles in the liquid flowing in the downstream flow channel 110 can be reduced. Therefore, a decrease in the cooling efficiency of the second light sources 72 can be reduced.

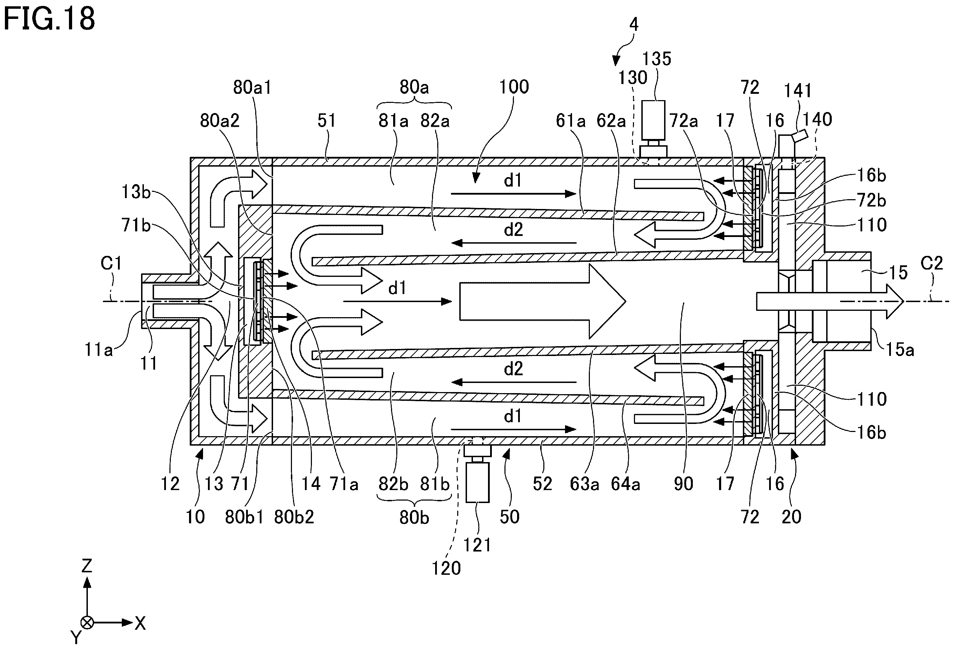

FIG. 18 is a cross-sectional view of an ultraviolet light fluid treatment device 4 according to the fourth embodiment of the present invention. As illustrated in FIG. 18, the ultraviolet light fluid treatment device 4 includes a first exhaust port 130, a first exhaust mechanism 135, a second exhaust port 140, and a second exhaust mechanism 141.

The first exhaust port 130 is a through hole formed in the first wall 51 located on the upper side in the vertical direction of the ultraviolet light fluid treatment device 4. The first exhaust port 130 can be opened and closed by a plug. In a state in which the plug of the first exhaust port 130 is opened, air bubbles in the liquid inside the ultraviolet light fluid treatment device 4 flow vertically upward by the action of buoyancy and are discharged through the first exhaust port 130. In a state in which the plug of the first exhaust port 130 is closed, air bubbles in the liquid inside the ultraviolet light fluid treatment device 4 are not discharged. The position where the first exhaust port 130 is formed is not particularly limited as long as the first exhaust port 130 is formed in the first wall 51.

The first exhaust mechanism 135 is a mechanism that can adjust the amount of air bubbles discharged from the first exhaust port 130. For example, the first exhaust mechanism 135 is a drain device including a plug that can adjust opening and closing of the first exhaust port 130. The amount of liquid discharged from the first exhaust port 130 can be adjusted by adjusting opening and closing of the plug of the ultraviolet light fluid treatment device 4. The first exhaust mechanism 135 is not an essential component; however, the ultraviolet light fluid treatment device 4 preferably includes the first exhaust mechanism 135 from the viewpoint of improving the workability of a drain operation.

The second exhaust port 140 is a through hole formed in the second end portion 20 on the upper side in the vertical direction of the ultraviolet light fluid treatment device 4. The second exhaust port 140 can be opened and closed by a plug. In a state in which the plug of the second exhaust port 140 is opened, air bubbles in the liquid inside the ultraviolet light fluid treatment device 4 flow vertically upward by the action of buoyancy and are discharged through the second exhaust port 140. In a state in which the plug of the second exhaust port 140 is closed, air bubbles in the liquid inside the ultraviolet light fluid treatment device 4 are not discharged. The position where the second exhaust port 140 is formed is not particularly limited as long as the second exhaust port 140 is formed in second end portion 20 on the upper side in the vertical direction.

The second exhaust mechanism 141 is a mechanism that can adjust the amount of air bubbles discharged from the second exhaust port 140. For example, the second exhaust mechanism 141 is a drain device including a plug that can adjust opening and closing of the second exhaust port 140. The amount of liquid discharged from the second exhaust port 140 can be adjusted by adjusting opening and closing of the plug of the ultraviolet light fluid treatment device 4. The second exhaust mechanism 141 is not an essential component; however, the ultraviolet light fluid treatment device 4 preferably includes the second exhaust mechanism 141 from the viewpoint of improving the workability of a drain operation.

As described above, in the present embodiment, the ultraviolet light fluid treatment device 4 includes the first exhaust port 130. Thus, air bubbles in the liquid filled in the device can be reduced. Accordingly, a decrease in the treatment efficiency of the ultraviolet light fluid treatment device 4 can be reduced. Further, in the present embodiment, the ultraviolet light fluid treatment device 4 includes the second exhaust port 140. Thus, air bubbles in the liquid flowing in the downstream flow channel 110 can be reduced. Accordingly, a decrease in the cooling efficiency of the second light sources 72 can be reduced.

### [Fifth Embodiment]

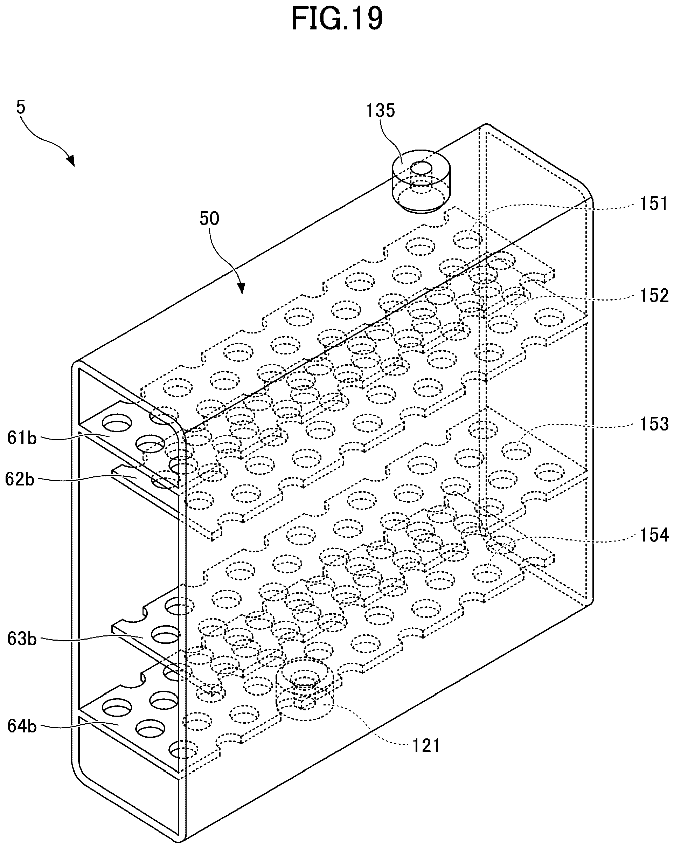

FIG. 19 is a drawing illustrating a plurality of partition members 61b to 64b included in an ultraviolet light fluid treatment device 5 according to a fifth embodiment of the present invention. FIG. 19 illustrates the intermediate portion 50 extracted from the ultraviolet light fluid treatment device 5. As the plurality of partition members 61a to 64a, for example, four partition members (a first partition member 61b, a second partition member 62b, a third partition member 63b, and a fourth partition member 64b) are disposed in the intermediate portion 50.

A plurality of first openings 151 is formed in the first partition member 61b. Each of the first openings 151 is a hole penetrating the first partition member 61b in the thickness direction of the first partition member 61b. A plurality of second openings 152 is formed in the second partition member 62b. Each of the second openings 152 is a hole penetrating the second partition member 62b in the thickness direction of the second partition member 62b. A plurality of third openings 153 is formed in the third partition member 63b. Each of the third openings 153 is a hole penetrating the third partition member 63b in the thickness direction of the third partition member 63b. A plurality of fourth openings 154 is formed in the fourth partition member 64b. Each of the fourth openings 15 is a hole penetrating the fourth partition member 64b in the thickness direction of the fourth partition member 64b. In the following, when the plurality of first openings 151, the plurality of second openings 152, the plurality of third openings 153, and the plurality of fourth openings 154 are not particularly distinguished, they are collectively referred to as openings 150.

In the present embodiment, the ultraviolet light fluid treatment device 5 has the openings 150. Thus, when liquid inside the device is discharged to empty the device, the liquid can easily flow vertically downward through the openings 150, thereby allowing the liquid to be less likely to remain in the device. Further, in the present embodiment, the ultraviolet light fluid treatment device 5 has the openings 150, and thus air bubbles in the liquid inside the device can easily move vertically upward through the openings 150. Accordingly, air bubbles in the liquid filled in the device can be reduced, and a decrease in the treatment efficiency of the ultraviolet light fluid treatment device 5 can be reduced.

The shape of the openings 150 in a top view is not particularly limited, and any shape such as a circular shape, an elliptical shape, a rectangular shape, or a polygonal shape can be appropriately selected. Further, the number, positions, intervals, and the like of the openings 150 can be appropriately selected. Openings 150 may be formed only in a portion of each of the first partition member 61b, the second partition member 62b, the third partition member 63b, and the fourth partition member 64b. With this configuration, in addition to effects of reducing residual liquid and reducing air bubbles as described above, an effect of smoothing the flow of fluid in the flow channel 100 of the ultraviolet light fluid treatment device 5, in other words, an effect of regulating the flow of the fluid can be obtained.

### [Sixth Embodiment]

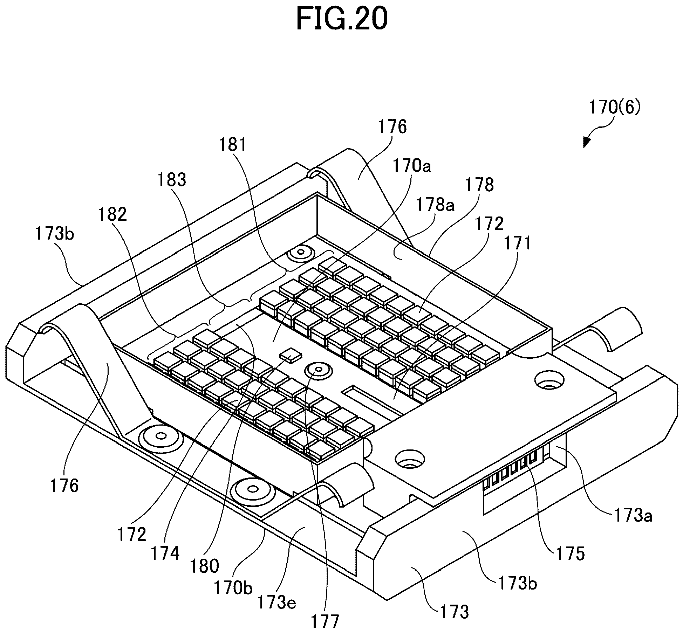

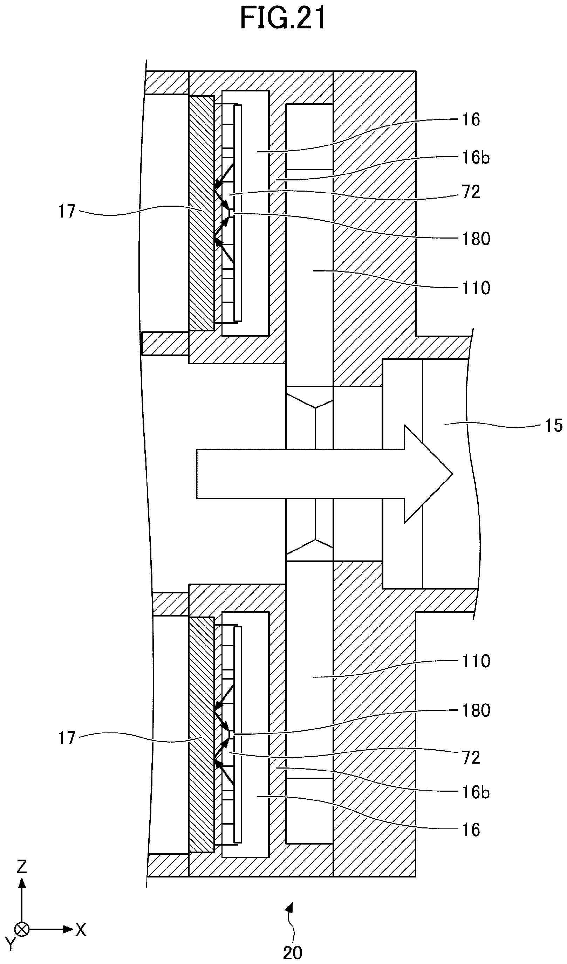

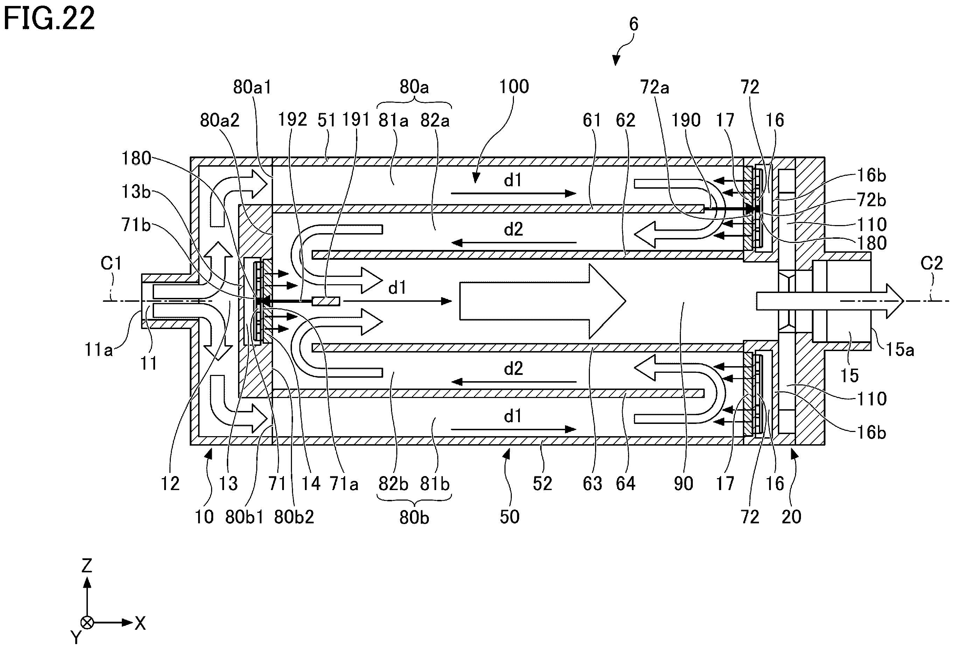

An ultraviolet light fluid treatment device 6 according to a sixth embodiment of the present invention will be described with reference to FIG. 20 through FIG. 22. FIG. 20 is a perspective view of a light source 170 including a photodetector 180 in the ultraviolet light fluid treatment device 6. FIG. 21 and FIG. 22 are drawings illustrating the operation of the photodetector 180. FIG. 21 is a first drawing and FIG. 22 is a second drawing.

As illustrated in FIG. 20, the ultraviolet light fluid treatment device 6 includes the photodetector 180. The photodetector 180 is provided on a first surface 170a of the light source 170. The installation position of the photodetector 180 can be appropriately changed as long as the photodetector 180 is provided within a region surrounded by a light-reflecting member 178.

The photodetector 180 includes a light receiving element that receives ultraviolet light, and outputs information corresponding to the light intensity of the received ultraviolet light to an external device. Examples of the light receiving element include a photoelectric conversion element such as a photodiode. For example, the photodetector 180 can output information corresponding to the light intensity of ultraviolet light having a wavelength of 265 nm, 280 nm, or the like. The output information from the photodetector 180 is a voltage signal, a current signal, or the like. The external device is a control device or the like that controls the operation of the ultraviolet light fluid treatment device 6.

As illustrated in FIG. 21, a portion of ultraviolet light, emitted from a second light source 72 of the two second light sources 72 toward the corresponding second window 17, is transmitted through the second window 17 and is emitted into the flow channel 100, and the other portion of the ultraviolet light is reflected by the second window 17 toward a photodetector 180. The photodetector 180 receives the ultraviolet light emitted from the second light source 72 and reflected by the second window 17. The photodetector 180 outputs information corresponding to the light intensity of the received ultraviolet light.

For example, there may be a case where the light intensity of the ultraviolet light emitted from the second light source 72 decreases due to deterioration of the second light source 72 according to a use period or the like of the second light source 72. If the light intensity of the ultraviolet light decreases, the treatment effect of the ultraviolet light fluid treatment device would decrease.

In the ultraviolet light fluid treatment device 6, if the second light source 72 deteriorates, output information from the photodetector 180 includes information indicating a decrease in the light intensity of the ultraviolet light emitted from the second light source 72. This output information allows the ultraviolet light fluid treatment device 6 to provide a notification indicating the deterioration of the second light source 72 to the external device. The external device can control the drive signal of the second light source 72 according to the degree of the deterioration of the second light source 72, and can notify a user or the like who uses the ultraviolet light fluid treatment device 6 of information prompting the user to replace the second light source 72.

The photodetector 180 provided on the second light source 72 is illustrated in FIG. 21; however, the same effects as those described above can be obtained by providing the photodetector 180 on the first light source 71.

The photodetector 180 can detect not only the deterioration of the light source 170 but also the state of contamination of the flow channel 100.

For example, in an ultraviolet light fluid treatment device, a portion of a flow channel may be contaminated due to adhesion of foreign matter to the surface of a window such as the second window 17. If the flow channel is contaminated, ultraviolet light emitted from a light source such as the second light source 72 toward fluid flowing in the flow channel would be blocked or absorbed by the contaminated portion, and as a result, the treatment effect of the ultraviolet light fluid treatment device would decrease.

As illustrated in FIG. 22, in the ultraviolet light fluid treatment device 6, a portion of the ultraviolet light emitted from the second light source 72 toward the second window 17 is transmitted through the second window 17 and is emitted into the flow channel 100. A portion of the emitted light is reflected by the end portion of the first partition member 61, is transmitted through the second window 17 from the flow channel 100 side to the second light source 72 side, and reaches the photodetector 180 provided on the second light source 72. Reflected light 190 indicated by an arrow represents the ultraviolet light reflected by the end portion of the first partition member 61.

In the ultraviolet light fluid treatment device 6, if the flow channel 100 is contaminated, output information from the photodetector 180 includes information indicating that the flow channel 100 is contaminated. This output information allows the ultraviolet light fluid treatment device 6 to provide a notification indicating the contamination of the flow channel 100 to the external device. Based on the output information from the ultraviolet light fluid treatment device 6, the external device can control the drive signal of the second light source 72 according to the degree of the contamination of the flow channel 100, and can notify the user or the like who uses the ultraviolet light fluid treatment device 6 of information prompting the user to clean the flow channel 100.

As illustrated in FIG. 22, the ultraviolet light fluid treatment device 6 may include a reflective member 191 in the merged flow channel 90, in order to detect contamination of the flow channel 100 due to adhesion of foreign matter to the surface of a first window 14. The shape, the position, the material, and the like of the reflective member 191 are not limited as long as the reflective member 191 can reflect the ultraviolet light; however, the reflective member 191 preferably has a small influence on the flow of the fluid in the merged flow channel 90.

A portion of the ultraviolet light, emitted from the first light source 71 toward the first window 14, is transmitted through the first window 14 and is emitted into the merged flow channel 90 in the flow channel 100. A portion of the ultraviolet light is reflected by the reflective member 191, is transmitted through the first window 14 from the flow channel 100 side to the first light source 71 side, and reaches a photodetector 180. Reflected light 192 indicated by an arrow represents the ultraviolet light reflected by the reflective member 191.

With the above configuration, the ultraviolet light fluid treatment device 6 can detect contamination of the flow channel 100 due to adhesion of foreign matter to the surface of the first window 14, and can output, to the external device, output information including information indicating that the flow channel 100 is contaminated. Based on the output information from the ultraviolet light fluid treatment device 6, the external device can control the drive signal of the first light source 71 according to the degree of the contamination of the flow channel 100, and can notify the user or the like who uses the ultraviolet light fluid treatment device 6 of information prompting the user to clean the flow channel 100.

Embodiments of the present invention have been described above with reference to specific examples. However, the present invention is not limited to the specific examples. Any embodiment that can be implemented by an appropriate design modification by a person skilled in the art based on the above-described embodiments of the present invention is also encompassed within the scope of the present invention as long as the features of the present invention are included. In addition, various changes and modifications can be conceived by a person skilled in the art within the spirit and scope of the present invention, and it is understood that the changes and modifications are also encompassed within the spirit and scope of the present invention.

For example, in each of the above-described embodiments, the fluid entering the merged flow channel 90 flows into each of the outlet 15 and the downstream flow channel 110; however, all of the fluid entering the merged flow channel 90 may flow into the downstream flow channel 110.

If the fluid entering the merged flow channel 90 flows into each of the outlet 15 and the downstream flow channel 110, there may be a case where the fluid does not reach the vicinity of each of the upper and lower ends in the vertical direction of the downstream flow channel 110. In the regions where the fluid does not reach, cooling effects of the second light sources 72 may be insufficient.

By allowing all of the fluid entering the merged flow channel 90 to flow into the downstream flow channel 110, the fluid can reach the vicinity of each of the upper and lower ends in the vertical direction of the downstream flow channel 110. Accordingly, cooling effects of the second light sources 72 can be appropriately obtained.

Aspects of the present invention are as follows, for example.
<1> An ultraviolet light fluid treatment device includes an inlet and an outlet of fluid; a flow channel connecting the inlet and the outlet and including a plurality of branch flow channels branching from the inlet, and a merged flow channel connected to a downstream side of each of the branch flow channels; a first light source configured to emit ultraviolet light to the merged flow channel; and a plurality of second light sources configured to emit the ultraviolet light to the plurality of respective branch flow channels.
<2> The ultraviolet light fluid treatment device according to <1>, wherein at least one of the plurality of branch flow channels includes a first flow channel disposed on an upstream side and a second flow channel disposed downstream of the first flow channel, and the first flow channel extends in a first direction, and the second flow channel extends in a direction different from the first direction.
<3> The ultraviolet light fluid treatment device according to <2>, wherein the second flow channel extends in a direction opposite to the first direction, and is disposed adjacent to the first flow channel.
<4> The ultraviolet light fluid treatment device according to <2> or <3>, wherein at least one of the plurality of second light sources is disposed at a position where the first flow channel and the second flow channel are irradiated with the ultraviolet light.
<5> The ultraviolet light fluid treatment device according to any one of <2> to <4>, further including a partition member configured to partition the first flow channel and the second flow channel, wherein each of the second light sources includes a wiring substrate, and a plurality of light emitting elements disposed on the wiring substrate, the wiring substrate has a first region in which light emitting elements of the plurality of light emitting elements are disposed, and that faces the first flow channel, a second region in which light emitting elements of the plurality of light emitting elements are disposed, and that faces the second flow channel, and a third region that is located between the first region and the second region, in which no light emitting element is disposed, and that faces the partition member.
<6> The ultraviolet light fluid treatment device according to any one of <1> to <5>, wherein at least two branch flow channels of the plurality of branch flow channels have a same cross-sectional shape orthogonal to a direction in which the fluid flows, and have a same length from one end to another end of each of the at least two branch flow channels.
<7> The ultraviolet light fluid treatment device according to any one of <1> to <6>, wherein a cross-sectional area of the merged flow channel is greater than a cross-sectional area of each of the branch flow channels, the cross-sectional areas each being orthogonal to a direction in which the fluid flows.
<8> The ultraviolet light fluid treatment device according to any one of <1> to <7>, wherein a cross-sectional area of at least one of the plurality of branch flow channels is greater than or equal to a cross-sectional area of the inlet, the cross-sectional areas each being orthogonal to a direction in which the fluid flows.
<9> The ultraviolet light fluid treatment device according to any one of <1> to <8>, wherein a cross-sectional shape of each of the inlet and the outlet orthogonal to a direction in which the fluid flows is a circular shape.
<10> The ultraviolet light fluid treatment device according to <9>, wherein a central axis of the inlet coincides with a central axis of the outlet.
<11> The ultraviolet light fluid treatment device according to any one of <1> to <10>, wherein a width of at least one of the plurality of branch flow channels differs from a width of any other branch flow channel of the plurality of branch flow channels, the widths each being in a direction orthogonal to a direction in which the fluid flows.

This application is based on and claims priority to Japanese Patent Application No. 2021-113658, filed on July 8, 2021, Japanese Patent Application No. 2021-152303, filed on September 17, 2021, Japanese Patent Application No. 2021-201185, filed on December 10, 2021, and Japanese Patent Application No. 2022-078359, filed on May 11, 2022, the entire contents of which are incorporated herein by reference.

### DESCRIPTION OF THE REFERENCE NUMERALS

1, 2, 3, 4, 5, 6 ultraviolet light fluid treatment device
10, 210 first end portion
11, 211 inlet
15, 215 outlet
20, 220 second end portion
50, 250 intermediate portion
71 first light source
72 second light source
80a, 80b, 280a, 280b branch flow channel
81a, 81b first flow channel
82a, 82b second flow channel
90, 290 merged flow channel
100 flow channel
170 light source
171 wiring substrate
172 housing including light emitting element
173 holding member
174 wiring
176 spring member
181 first region
182 second region
183 third region
270 light source
271 wiring substrate
272 housing including light emitting element
273 holding member
274 wiring
276 spring member
281 first region
282 second region
283 third region
286 cover glass
291 waterproof cap
91 end portion
92, 97 flow
93a1, 93a2, 93b1, 93b2 turning back portion
94a1, 94a2, 94b1, 94b2 vortex
96 vortex
95 merging portion
98 flow
θ1, θ2, θ3, θ4 angle
120 drain port
121 drain mechanism
131 first downstream end
132 second downstream end
133 third downstream end
134 fourth downstream end
130 first exhaust port
135 first exhaust mechanism
140 second exhaust port
141 second exhaust mechanism
150 opening
151 first opening
152 second opening
153 third opening
154 fourth opening
180 photodetector
190, 192 reflected light
191 reflective member

## Claims

1. An ultraviolet light fluid treatment device comprising:
an inlet and an outlet of fluid;
a flow channel connecting the inlet and the outlet and including
a plurality of branch flow channels branching from the inlet, and
a merged flow channel connected to a downstream side of each of the branch flow channels;
a first light source configured to emit ultraviolet light to the merged flow channel; and
a plurality of second light sources configured to emit the ultraviolet light to the plurality of respective branch flow channels.

2. The ultraviolet light fluid treatment device according to claim 1, wherein at least one of the plurality of branch flow channels includes a first flow channel disposed on an upstream side and a second flow channel disposed downstream of the first flow channel, and
the first flow channel extends in a first direction, and the second flow channel extends in a direction different from the first direction.

3. The ultraviolet light fluid treatment device according to claim 2, wherein the second flow channel extends in a direction opposite to the first direction, and is disposed adjacent to the first flow channel.

4. The ultraviolet light fluid treatment device according to claim 2 or 3, wherein at least one of the plurality of second light sources is disposed at a position where the first flow channel and the second flow channel are irradiated with the ultraviolet light.

5. The ultraviolet light fluid treatment device according to any one of claims 2 to 4, further comprising:
a partition member configured to partition the first flow channel and the second flow channel,
wherein each of the second light sources includes
a wiring substrate, and
a plurality of light emitting elements disposed on the wiring substrate,
the wiring substrate has
a first region in which light emitting elements of the plurality of light emitting elements are disposed, and that faces the first flow channel,
a second region in which light emitting elements of the plurality of light emitting elements are disposed, and that faces the second flow channel, and
a third region that is located between the first region and the second region, in which no light emitting element is disposed, and that faces the partition member.

6. The ultraviolet light fluid treatment device according to any one of claims 1 to 5, wherein at least two branch flow channels of the plurality of branch flow channels have a same cross-sectional shape orthogonal to a direction in which the fluid flows, and have a same length from one end to another end of each of the at least two branch flow channels.

7. The ultraviolet light fluid treatment device according to any one of claims 1 to 6, wherein a cross-sectional area of the merged flow channel is greater than a cross-sectional area of each of the branch flow channels, the cross-sectional areas each being orthogonal to a direction in which the fluid flows.

8. The ultraviolet light fluid treatment device according to any one of claims 1 to 7, wherein a cross-sectional area of at least one of the plurality of branch flow channels is greater than or equal to a cross-sectional area of the inlet, the cross-sectional areas each being orthogonal to a direction in which the fluid flows.

9. The ultraviolet light fluid treatment device according to any one of claims 1 to 8, wherein a cross-sectional shape of each of the inlet and the outlet orthogonal to a direction in which the fluid flows is a circular shape.

10. The ultraviolet light fluid treatment device according to claim 9, wherein a central axis of the inlet coincides with a central axis of the outlet.

11. The ultraviolet light fluid treatment device according to any one of claims 1 to 10, wherein a width of at least one of the plurality of branch flow channels differs from a width of any other branch flow channel of the plurality of branch flow channels, the widths each being in a direction orthogonal to a direction in which the fluid flows.
